# EUROPEAN PATENT SPECIFICATION

(11) **EP 1 503 661 B1**
(45) Date of publication and mention of the grant of the patent: **24.09.2008**
(21) Application number: 03734037.9
(22) Date of filing: 02.05.2003
(51) Int. Cl.: A61B 5/00, G01N 33/49, A01N 1/02, A61B 17/00, G01N 27/416, G01N 33/487, A61B 5/042, A61M 1/36, A61M 5/172

(54) **SYSTEMS OF PH TISSUE MONITORING**
SYSTEME ZUR GEWEBE-PH-MESSUNG
SYSTEMES DE SUIVI DE PH TISSULAIRE

(30) Priority: 02.05.2002 US 137709
(43) Date of publication of application: 09.02.2005
(73) Proprietor: Surgical Technology Advancements, LLC, Westwood, MA 02090 (US)
(72) Inventor: KHURI, Shukri, F., Westwood, MA 02090 (US); TREANOR, Patrick, Dedham, MA 02026 (US)
(74) Representative: Gambell, Derek
(86) International application number: PCT/US2003/015328
(87) International publication number: WO 2003/092489

(56) References cited:
- WO-A-99/08589
- FR-A- 2 744 804
- US-A- 4 562 846
- US-A- 4 717 548
- US-A- 5 573 502
- US-A- 5 588 816
- US-A- 5 899 867

## Description

### BACKGROUND OF THE INVENTION

It is well known in the art to determine the pH in body fluids by using an electrode cell assembly and immersing the measuring electrode into a sample of the bodily fluid. The pH is known to be the symbol for the negative logarithm of the H⁺ ion concentration. The pH value of the blood indicates the level of acidity of the blood. High blood acidity, which is reflected by a low pH indicates, in that the organs of the body are not being provided with enough oxygen, which can ultimately prove harmful.

It is also known in the art to measure tissue pH in myocardial tissue. Measurement of pH in myocardial tissue has been used to determine the presence of myocardial ischemia, as indicated by tissue acidosis which is reflected by a decrease in pH. During cardiac surgery, the aorta is cross clamped and the myocardium is deprived of its blood and nutrient supply, creating the potential for damage to the heart from ischemia. Ischemia can be diagnosed by monitoring the pH of the myocardium which falls significantly and becomes acidotic during ischemia.

Multiple methods have been suggested for the measurement of myocardial tissue hydrogen ion [H⁺] or pH. Epicardial (surface) electrodes were used in the early 1970s but were abandoned because of a lack of reproducibility and failure to measure pH in the deeper, more vulnerable layers of the myocardium. Plunge electrodes with glass or polymeric tips as well as fiberoptic probes have been used with variable results.

Further, phosphorus-31 nuclear magnetic resonance (NMR) spectroscopy and fluorescent imaging techniques allow the measurement of intracellular myocardial pH, and are commonly used in a variety of cell, organ, and animal preparations. These techniques, however, are still not applicable to the operating room setting.

Cardiac surgery is a major potential source of injury because of its complexity, the wide variation in severity of illness of the patients who undergo it, and the need to interrupt the blood supply to the heart in the course of surgery. The typical gauge to determine safe surgery is the post-operative outcome of the patient. Document US-A-4 562 846 discloses a tissue monitoring system for assessing the myocardial status during surgery. There is an ongoing need, however, for systems that can be used during the operation to diagnose, gauge, and prevent adverse ischemic damage to the heart.

### SUMMARY OF THE INVENTION

While ischemia or tissue acidosis, in cardiac tissue has been measured, systems to prevent and/or reverse tissue, and in particular, cardiac acidosis were unknown, particularly in the face of wide swings in tissue temperature. Surgeons did not know how to measure tissue acidosis in the face of changes in temperature and to reverse tissue acidosis once discovered. The present invention relates to systems of using tissue pH measurements to diagnose ischemia and to gauge the conduct of an operation, based on these pH measurements, so as to prevent and/or reverse tissue ischemia/acidosis. The present invention is disclosed in claim 1 with preferred embodiments in the dependent claims.

The present invention relates to pH-guided management of tissue ischemia or the use of pH measurements of tissue corrected for temperature, as a system for controlling diagnostic and/or surgical procedures. A preferred embodiment of the invention relates specifically to an apparatus which is applicable to patients undergoing cardiac surgery. It employs a tissue electrode (which measures both pH and temperature) and a monitor and comprises a series of steps that, in a preferred embodiment, are aimed at achieving a homogeneous and effective distribution of cardioplegic solution during aortic clamping, and at insuring adequate revascularization of ischemic segments of the myocardium. A method using pH-guided myocardial management guides the conduct of operations, prevents damage to the heart, extends the safe period of oxygen deprivation, and improves the outcome of patients undergoing heart surgery.

The use of the pH-guided myocardial management system to identify ischemic segments of the myocardium can provide a user with options for specific courses of conduct, both during and after, the surgical procedure. These options include: effecting an optimal delivery of preservation solutions to the heart to reduce ischemia, assessing the adequacy of coronary revascularization following a heart surgery procedure, identifying viable but nonfunctioning heart muscle that would benefit from revascularization, prompting changes in the conduct of the surgical procedure, monitoring the pH of the heart muscle post-operatively and evaluating the efficacy of newer myocardial protective agents.

There are several methods of delivery of a pH electrode, used in pH-guided myocardial management, to a site of interest. The electrode can be delivered manually by the user. The electrode can also be delivered by a catheter through a percutaneous incision. The electrode can also be delivered by an endoscope, a colonscope or a laparoscope to a site of interest.

Other systems can also be used to measure pH, including, in certain applications, surface pH measurements, magnetic resonance measurements, or optical methods using fiber optic probes or endoscopes.

When a user has found that tissue acidosis is present at a site of interest, the user can effect an optimal delivery of preservation fluids, or cardioplegia fluids, to the heart to raise the pH of the site. Several systems and techniques that provide optimal delivery of the cardioplegia solutions to the site are available to the user. These include: altering the flow rate of the preservation fluid, altering the temperature of the fluid, altering the site of delivery, repositioning the tip of the catheter, selectively directing the preservation fluid through the manifold, applying direct coronary artery pressure on the proximal portion of the artery, occluding the left main coronary artery with a balloon catheter, inflating/deflating the balloon of a retrograde coronary sinus catheter, administering a bolus of cardioplegia through the orifice of a right coronary artery and directing cardioplegia through specific previously constructed grafts.

When a user has found that tissue acidosis is present at a site of interest, the user can also prompt changes to the conduct of the surgical procedure to raise the pH of the site. Several alternatives for changing the surgical procedure are available to the user. These include: determining the need for revascularization of a specific segment of the myocardium, changing the order of revascularization, providing additional revascularization, changing the operation or the surgeon to reduce ischemic time, canceling an operation and delaying the weaning of a patient from cardiopulmonary bypass.

The pH electrode itself can have a cable connected to a silver wire where the silver wire is an Ag/AgCl (silver/silver chloride) wire. The cable and wires are encased in a housing which is encased in shrink tubing. The electrode has a glass stem which houses the silver wire, a thermistor (i.e. a temperature sensor), a pH sensor, and a gelled electrolyte. The electrode has a bendable joint which allows the user to adjust the positioning of the electrode prior to or during use and which facilitates electrode removal after chronic insertion. The glass stem is pointed to allow direct insertion into tissues. In a preferred embodiment, the glass stem is made of lead glass.

The electrodes can be used in a probe that can be delivered to a site within the human body using a catheter and/or endoscope. The sensor can be connected to a data processing system such as a personal computer that can be used to record and process data.. The computer can be programmed using a software module to correct the pH for temperature control system operation and to indicate to the user the status of the patient and changes in system status and operation. The system can also prompt the surgeon as to indicated changes in a surgical procedure in progress. The computer can be connected to a controller that operates a fluid delivery system and various temperature and pressure sensors can provide data for the monitoring system regarding patient status.

Another advantage of the present invention includes online, real-time measurement and monitoring of myocardial tissue pH. In particular, myocardial tissue pH is monitored in the anterior and posterior walls of the left ventricle in patients undergoing cardiac surgery. In accordance with one aspect of the present invention, the relationship between pH and temperature and between the pH and the hydrogen ion [H⁺] in tissues is used for interpreting the myocardial pH data generated in the course of a cardiac surgical procedure. Intraoperative monitoring of myocardial pH is an important modality for the surgeon for online assessment and improvement of the adequacy of myocardial protection. Myocardial protection is correlated to protection from myocardial tissue acidosis. This measure of acidosis also provides an online tool for assessing the adequacy of coronary revascularization and outcomes for off-pump coronary artery bypass grafting.

In a preferred embodiment an improved 30-day and long-term postoperative outcome is achieved when the integrated mean pH during the period of aortic clamping is kept at or above pH 6.8 in both the anterior and posterior walls of the left ventricle. In a preferred embodiment, myocardial pH, when measured simultaneously in the anterior and posterior walls of the left ventricle provides a reliable presentation of the global acid-base balance in the left ventricular myocardium.

A method of detecting acidosis in tissue comprises the steps of contacting the tissue of a patient with a first pH electrode disposed in an anterior wall of the left ventricle, and further contacting the tissue of the patient with a second pH electrode disposed in a posterior wall of the left ventricle. The method includes measuring the pH of the tissue with the first and second electrode during cardiac surgery, monitoring the pH of the tissue with the first and second electrode during cardiac surgery, determining if the tissue pH falls below a threshold level indicative of acidosis with the first and second electrode, and determining a post-operative outcome based on the threshold level and the tissue pH measured by the first and second electrode. In a preferred embodiment the normal and temperature corrected tissue pH range is 7.10-7.30, mild moderate acidosis ranges from 6.69-6.50, severe acidosis ranges from 6.49-6.20, very severe acidosis is pH ≤ 6.19.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
Figure 1 illustrates a method of using tissue pH to identify ischemic segments of a myocardium and the options available to a user to utilize this information and take an appropriate course of action.
Figure 2 illustrates the methods of delivery of a pH electrode to cardiac tissue.
Figure 3 illustrates a method of effecting an optimal delivery of preservation solution to the heart during surgery.
Figure 4 illustrates a method of using the pH electrode to measure the condition of tissue and alter the conduct of an operation involving the tissue.
Figure 5 illustrates a sectional view of a preferred embodiment of a pH electrode.
Figure 6 illustrates a turkey foot configuration of a cardioplegia delivery system and tools.
Figure 7A shows a manifold cardioplegia delivery system and tools attached to a heart.
Figure 7B shows a cannula placed within the left main coronary artery of the heart.
Figure 8 shows a coronary sinus cannula connected to a venous cannula.
Figure 9 graphically illustrates the pH monitored at the anterior and posterior wall of the left ventricle.
Figures 10A and 10B are graphical illustrations of the relationship between tissue pH measured with an electrode of a preferred embodiment and nuclear magnetic resonance (NMR) spectroscopy, respectively, during coronary artery occlusion.
Figure 11 is a graphical illustration of an anterior left ventricular wall pH and temperature recorded in the course of an aortic valve replacement, wherein the broken line shows myocardial temperature, which fell to 13°C with administration of cold cardioplegia delivered immediately after aortic clamping, the thin solid line shows actual myocardial pH whereas the thick solid line shows pH corrected to 37°C and XC = cross-clamp.
Figure 12 illustrates the relationship between pH (in units) and corresponding hydrogen ions in nanomoles.
Figure 13 graphically illustrates the myocardial pH recordings from anterior and posterior left ventricle walls in a 52-year-old man who underwent coronary artery bypass grafting to the left anterior descending coronary artery and aortic valve replacement. The site, duration and rate of delivery of the blood cardioplegia solution are illustrated.
Figure 14 graphically illustrates the temperature corrected myocardial pH recordings in a 67-year-old man undergoing complex aortic valve replacement. The pH is measured from three sites: anterior left ventricular (LV) wall (thick solid line); posterior LV wall (thick broken line); and anterior right ventricular (RV) wall (thin solid line).
Figure 15 graphically illustrates the response of anterior and posterolateral wall pH to rapid atrial pacing, wherein the bars on top show pacing rate.
Figure 16 illustrates the front panel of the user interface monitor with numerical values displayed on the screen.
Figure 17 illustrates a back view of a monitor included in the preferred embodiment of the pH monitoring system.
Figure 18 illustrates a view of a junction box.
Figure 19 illustrates the sensing end of the sensor used in the pH monitoring system.
Figure 20 illustrates the attachment of a pole clamp and the user interface monitor in the pH monitoring system.
Figure 21 is a display screen of a user interface monitor with which a user interfaces to monitor the pH of a system.
Figure 22 is a display screen of a user interface monitor with which a user interfaces to use a previous calibration.
Figure 23 illustrates a display screen with which a user interfaces if the junction box is not connected properly.
Figure 24 illustrates a display screen with which a user interfaces.
Figure 25 illustrates the display screen with which a user interfaces to choose general options for the setup.
Figure 26 illustrates the display screen with which a user interfaces to select and set printer options.
Figure 27 illustrates the display screen with which a user interfaces to select and set alerts.
Figure 28 illustrates the display screen with which a user interfaces to select and set graphic display options.
Figure 29 illustrates the display screen with which a user interfaces to select and set parameters for communicating with an external device.
Figure 30 is a view illustrating the sensor being calibrated for use in the pH monitoring system.
Figure 31 illustrates the display screen with which a user interfaces to set up the calibration of a sensor in the pH monitoring system.
Figure 32 illustrates the display screen with which a user interfaces to monitor the parameters such as pH and temperature during cardiac surgery.
Figure 33 graphically illustrates the survival curve for the baseline pH which is defined as the pH measured just prior to insertion of an aortic clamp.
Figure 34 graphically illustrates the survival curve for the end of the reperfusion pH.
Figure 35 graphically illustrates survival curves for different pH categories.

### DETAILED DESCRIPTION OF THE INVENTION

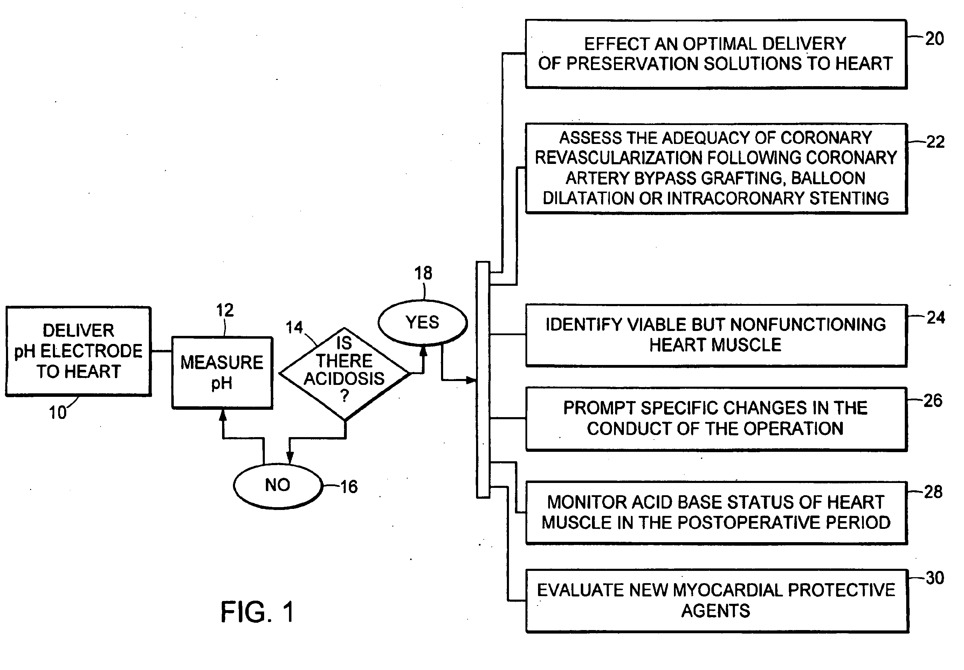

Figure 1 illustrates a method of using tissue pH to identify ischemic segments of the heart, which are regions of the heart muscle that are not receiving an adequate blood and nutrient supply, and the options available to a user to take advantage of this information and pursue an appropriate course of action. A user first delivers a pH electrode to a patient's heart 10. The user then measures the tissue pH as displayed on a monitor 12 and determine whether or not there is acidosis present in the tissue 14. If there is no tissue acidosis 16, the pH is again measured 12. In a preferred embodiment, the pH is continually measured by the electrode with the pH measurements displayed on a monitor. If acidosis existed in the tissue 18, however, the user uses this information to take appropriate action such as, but not limited to, the following actions.

A user can effect an optimal delivery of the preservation solutions to the heart through one or more of a compendium of specific interventions 20. To perform open heart surgery, the aorta has to be clamped thus depriving the heart muscle from its blood, nutrient, and oxygen supply. A preservation solution, often referred to as a cardioplegic solution, is normally perfused into the heart and its blood vessels to prevent time-dependent ischemic damage. It has been shown that the measurement of tissue pH, which reflects, in part, the washout of the hydrogen ion generated by the metabolic processes, is a good indicator of the regional distribution of the preservation solution. It has also been shown this distribution to be markedly heterogeneous and unpredictable, with segments of the myocardial wall suffering from acidosis because of failure of the cardioplegic solution to reach these segments. The main objective of pH-guided myocardial management is to prevent tissue acidosis in all the segments of the myocardium throughout the course of open heart surgery. This is achieved by insuring an adequate and a homogeneous delivery of the cardioplegic solution and an adequate revascularization of ischemic segments of the heart. These are achieved by maintenance of the myocardial pH as near normal as possible, with normal pH ranging between, in particular 7.1 through 7.3.

A user can also assess the adequacy of coronary revascularization following coronary artery bypass grafting, balloon dilatation or intracoronary stenting 22. This functionality employs the rate of washout of the hydrogen ion accumulating in the tissues during ischemia as an indication of the magnitude of tissue blood flow. Following restoration of flow through a newly constructed aorto-coronary bypass graft, no change in the pH of a myocardial segment subtended by that graft indicates inadequate revascularization. On the other hand, a rise in the pH of more than 0.1 pH units indicates restoration of effective tissue flow to the ischemic myocardium.

A user can also identify viable but non-functioning heart muscle 24, known as hibernating myocardium, which improves its function with adequate coronary revascularization. pH-guided myocardial management has demonstrated that the ability of the non-contractile myocardial wall segment to produce acid, i.e. to exhibit tissue acidosis, is an indication of the viability and reversibility of dysfunction in this segment. Hence the procedure provides a tool with which the viability of the non-contractile myocardial segment can be assessed.

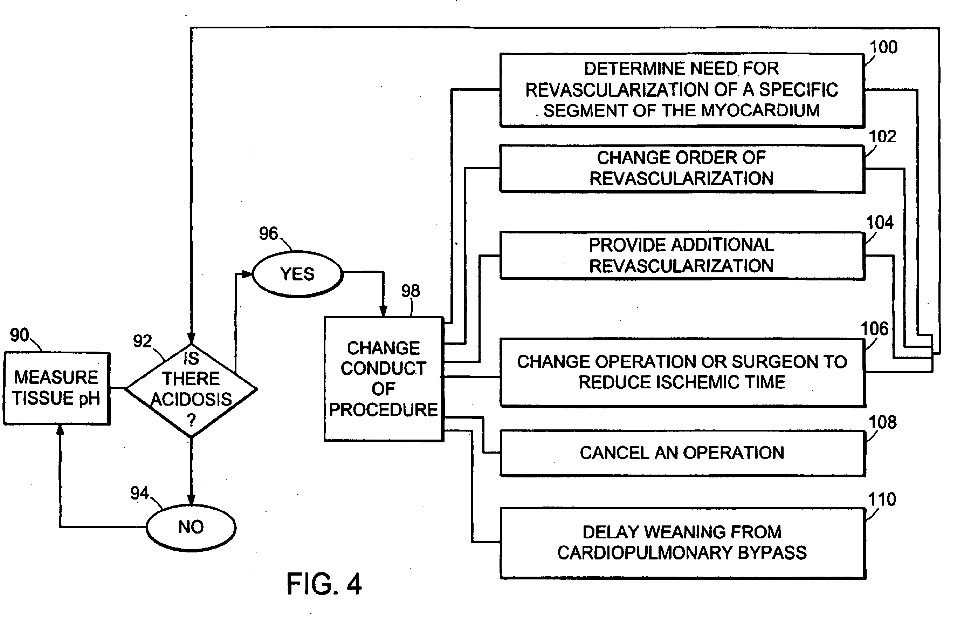

A user can also prompt specific changes in the conduct of the operation 26 after obtaining information regarding tissue pH. These changes in operating procedure are outlined in greater detail in Figure 4.

A user can also monitor the acid-base status of the heart muscle in the post-operative period 28 and identify impending problems. This functionality allows the depiction of ischemic events in the intensive care unit within the first 72 hours postoperatively. This methodology is capable of continuous monitoring of regional tissue metabolism and acid base balance in a patient, post-surgery. A fall in the myocardial pH of more than 0.1 pH units in the face of a stable blood pH is indicative of myocardial acidosis. The more severe the fall in the pH the more reflective of the extent of ischemia. This functionality is achieved by implanting the electrodes in the myocardium at the time of the operation and exteriorizing them through a special chest tube. The electrodes are pulled out in the surgical intensive care unit (SICU) after the monitoring is terminated by simply pulling on them along with the chest tube which houses them.

The user can also evaluate the efficacy of newer myocardial protective agents and methods in the prevention of tissue acidosis and the improvement of patient outcomes 30. To improve myocardial protection, a number of agents are being proposed as additions to the cardioplegic solution, and new modalities for the administration of cardioplegia are being sought. pH-guided myocardial management provides a metabolic marker which can enable the comparative assessment of the efficacy of these new agents and modalities in improving the degree of intraoperative protection, the hallmark of which can be the degree of prevention of acidosis during the periods of aortic clamping and reperfusion. The variable employed to compare these methods of myocardial protection is the integrated mean myocardial pH during the period of aortic clamping or during specific periods of reperfusion. The higher the integrated mean pH during these periods, the better is the degree of myocardial protection.

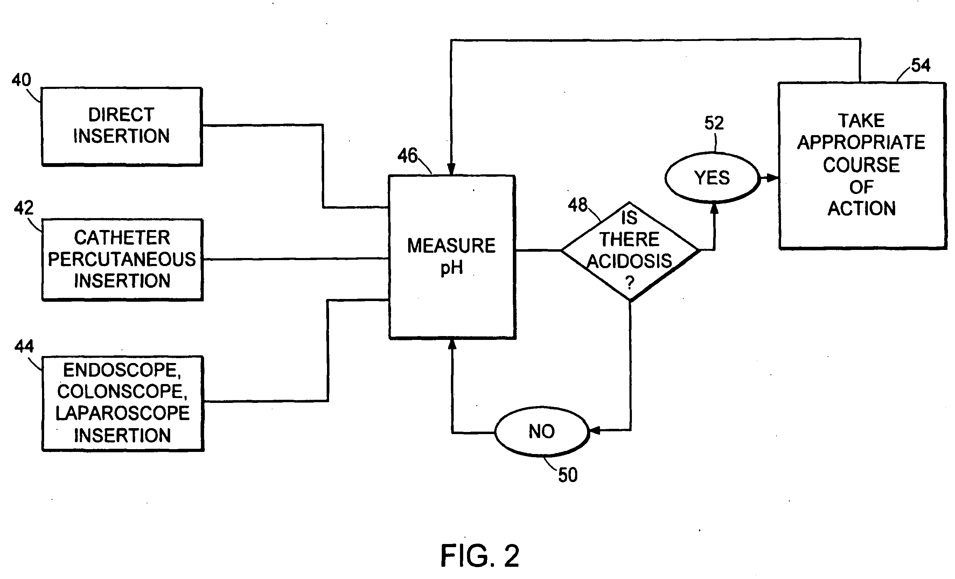

Figure 2 illustrates various methods of delivery of a pH electrode to cardiac tissue. A user can implant the pH electrode using direct insertion 40. This can include opening the chest cavity of a patient during a cardiac surgery procedure and placing the electrode into the patient's cardiac tissue by hand. The user can also insert the pH electrode by means of a catheter using a percutaneous incision 42. A user can also insert the pH electrode by using an endoscope, colonscope or laparoscope 44. The user can then measure the pH of the tissue 46 and determine whether there is acidosis in the tissue 48. If no acidosis is found 50, the pH of the tissue can again be measured 46. If acidosis is found in the tissue 52, the user can then take an appropriate course of action 54, as outlined in Figure 1.

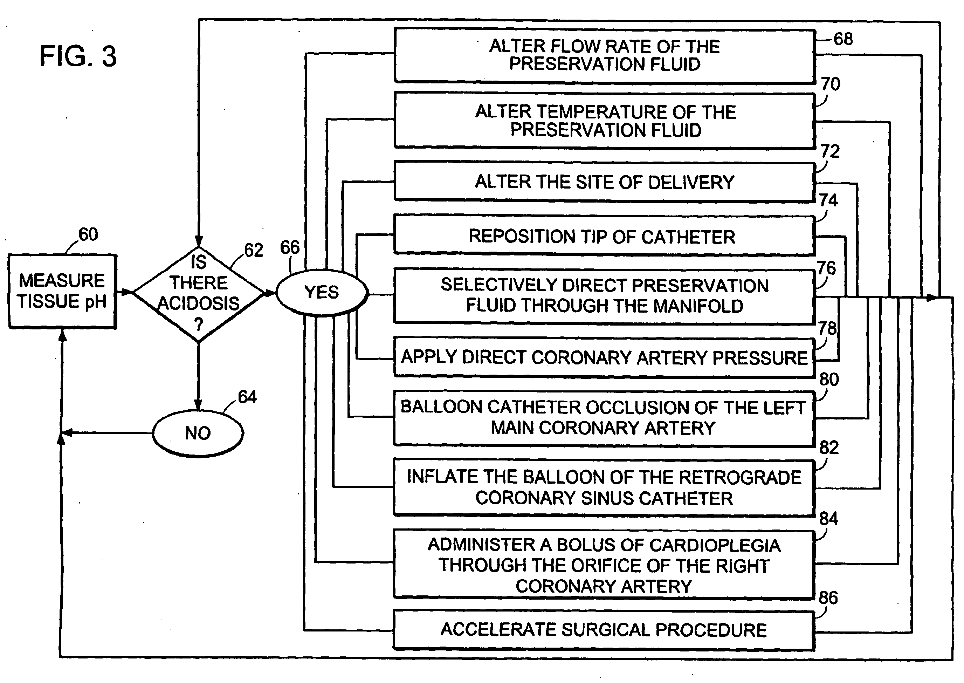

Figure 3 illustrates a method of providing for an optimal delivery of preservation solution to a heart during surgery. In this method, a user can first measure cardiac tissue pH 60 and determine whether there is acidosis in the tissue 62. If no acidosis is found 64, the pH of the tissue can again be measured 62. In a preferred embodiment, the pH is continuously measured and monitored. If acidosis is found in the tissue 66, the user can then effect an optimal delivery of the preservation solutions to the heart through one or more of a compendium of specific interventions. Interventions to be used to effect an adequate and a homogeneous delivery of the cardioplegic solution including, but are not limited, to the following maneuvers. The user can alter the flow rate of the preservation solution 68 to provide an optimal delivery of the cardioplegia solution. The perfusionist controls the flow rate of the cardioplegic solution administered. pH-guided myocardial management has demonstrated that patients and myocardial segments differ in the flow rate necessary to prevent acidosis. Therefore, changing the flow rate of the cardioplegia solution can alter and improve tissue pH.

The user can also alter the temperature of the preservation solution 70 to optimize solution delivery. Changes in myocardial temperature, which can range widely in the course of cardiac surgery, effect various degrees of vasoconstriction and vasodilatation of the coronary vasculature. This, in turn, effects the distribution of the cardioplegic solution and also the level of tissue acidosis. Avoidance of tissue acidosis can be achieved either by cooling or by re-warming the cardioplegic solution, depending on the effect of temperature on the regional distribution of the cardioplegic solution. pH-guided myocardial management has demonstrated that the effect of temperature on the regional distribution of the cardioplegic solution is totally unpredictable and, hence, continuous monitoring of myocardial tissue pH allows the determination of the myocardial temperature which is most likely to prevent myocardial acidosis. Opposite effects on myocardial pH have been observed from patient to patient with both cooling and rewarming. In general, however, giving warm cardioplegia effected an improvement in tissue pH in most patients.

To provide an optimal delivery of the solution, the user can also alter the site of delivery of the cardioplegic solution 72. The cardioplegic solution can be delivered through several sites: antegrade through the aortic root, antegrade through the orifice of the right and/or left main coronary arteries, antegrade through the proximal ends of newly constructed grafts, and retrograde through the coronary sinus. pH-guided myocardial management allows the surgeon to choose the site or combination of sites of administration which can best avoid regional acidosis.

The user can reposition the tip of the catheter through which the cardioplegic solution is delivered 74 to optimize delivery. This may need to be performed in patients with a very short left main coronary artery when cardioplegia is administered through the orifice of the left main. It can also be useful in pulling back on a retrograde catheter which is pushed too far into the coronary sinus.

The user can also selectively direct the cardioplegic solution through a manifold so as to reduce the steal of the solution 76. The cardioplegic solution can be delivered through a manifold having several catheters radiating from a single source. This arrangement of the manifold is known as a "turkey foot". When the cardioplegic solution is administered through more than one of these catheters simultaneously, there is a marked heterogeneity in the distribution of the solution to the various myocardial segments supplied by these catheters. The solution often moves preferentially into the catheter supplying the myocardial segment with least resistance, usually the myocardial segment with least coronary artery disease. This is what is referred to as a "steal phenomenon." Monitoring myocardial pH, which capitalizes on the fact that the rate of washout of the hydrogen ion in tissue is indicative of the magnitude of tissue flow, can determine which segments of the myocardium are receiving the cardioplegic solution and which segments are deprived of cardioplegia because of the "steal" phenomenon. When steal is encountered, homogeneity of the distribution of the cardioplegic solution can be achieved by occluding the catheters responsible for the steal and by specifically directing the flow only into the areas exhibiting acidosis.

The user can also apply direct coronary artery pressure on the proximal portion of the artery to distally direct cardioplegia flow through a newly constructed graft 78. This pressure can prevent the solution from going proximally preferentially, and forces the cardioplegia solution distally to an area with low pH, to lower tissue acidosis in that area.

The user can perform a balloon catheter occlusion of the orifice of the left main coronary artery during the delivery of retrograde cardioplegia through the coronary sinus or through the proximal ends of recently constructed saphenous vein grafts 80. The balloon catheter occlusion of the left main coronary artery prevents the steal phenomenon, where the solution follows the path of least resistance, and forces the cardioplegia solution to an area of low pH. This process can reverse acidosis of an area showing a low pH.

The user can also inflate the balloon of a retrograde coronary sinus catheter while the cardioplegic solution is being administered antegrade 82. Normally, if cardioplegia is being delivered antegrade and retrograde simultaneously, the balloon in the coronary sinus is kept deflated. A more homogeneous distribution of the cardioplegic solution can be achieved if the balloon in the coronary sinus is kept inflated while the cardioplegia is delivered simultaneously antegrade and retrograde.

The user can also administer a bolus of cardioplegia through the orifice of the right coronary artery when the latter is a dominant, non-obstructed vessel 84. In the course of an open heart operation in which the aortic root is open, cardioplegia can be administered through the orifice of the right coronary artery in addition to the orifice of the left coronary artery. This, however, can be tedious and time consuming, hence it is not a common practice. pH-guided myocardial management has shown that the posterior left ventricular wall is more vulnerable to refractory myocardial acidosis if the right coronary artery is dominant and no cardioplegia is administered through it. Hence, if in the course of pH-guided myocardial management, refractory acidosis is encountered in the posterior wall, administering a bolus of cardioplegia through the orifice of the right coronary artery, if the latter is dominant, can insure adequate delivery of the cardioplegic solution to the posterior wall and can reverse the acidosis.

A user can also accelerate the surgical procedure 86 when tissue acidosis is present. By monitoring tissue acidosis, a user can avoid either using his time wastefully or attempting nonstandard or potentially ineffectual surgical procedures. Also, in few patients, less than 5%, there is no known method to prevent tissue acidosis and the surgical procedure must be accelerated. With the acceleration of a procedure, the aorta, which is clamped during the surgery, is unclamped sooner than planned, thus allowing oxygen rich blood to reach the heart muscle, thereby reversing acidosis.

In the event that one of the described options, 68 through 86, fails to relieve the ischemic condition, as evidenced by the display of tissue pH levels on the pH monitor, the user can use any of the other described options to attempt to raise tissue pH.

Figure 4 illustrates a method of using the pH electrode to prompt specific changes in the conduct of an operation after determining there is tissue acidosis. In this method, a user first measures cardiac tissue pH 90 and determines whether there is acidosis in the tissue 92. If no acidosis is found 94, the pH of the tissue can again be continuously or periodically measured 90. If acidosis is found in the tissue 96, the user can then change the conduct of the procedure 98.

These changes can include, but are not limited, to the following maneuvers. First, the need for the revascularization of a specific segment of myocardium 100 is determined. The ability to identify which specifically are the segments of the myocardium that need revascularization can be lifesaving. Segments requiring revascularizaton can be determined by either examining the onset of regional acidosis in the course of an operation or the response of the myocardial pH to atrial pacing. The response to atrial pacing can be utilized intra-operatively, postoperatively in the SICU, and in the cardiac catheterization laboratory. A fall of 0.1 pH or more in a tissue segment in response to atrial pacing indicates a physiologically significant obstruction in the coronary artery subtending that segment, and hence the need to revascularize that coronary artery.

The user can also change the order of revascularization 102. pH-guided myocardial management allows the surgeon to revascularize the most ischemic segments of the myocardium first and to perfuse a cardioplegic solution through them so as to minimize the degree of acidosis encountered in the course of aortic clamping.

The user can also change the procedure by providing additional revascularization of the heart 104 base on unexpected acidosis encountered in the course of the operation such as may happen secondary to embolization of particular material into the coronary artery. pH-guided myocardial management involves identifying ischemic segments of the left ventricular wall that require revascularization, often unplanned preoperatively.

The user can also alter the plan of the operation or change the surgeon to reduce the duration of the ischemic time 106. pH-guided myocardial management allows for reductions in the magnitude of the planned operation in several ways. When pH monitoring depicts a significant amount of myocardial acidosis which cannot be corrected, the need to reduce the ischemic time becomes more important than the potential benefits of certain parts of the operation that can be dispensed with, such as the construction of an additional graft. pH monitoring also allows the surgeon to abandon a planned part of the operation because it uncovers no real need for this part. In this context, pH-guided myocardial management also plays a major value in the teaching of residents because it provides the attending surgeon with the information on what parts of the operation he/she can give to the resident, and what part the attending surgeon can be doing himself/herself, since residents, particularly early in their training, can be fairly tardy in performing these operations. The user can also cancel an operation 108 if, based on the pH measurements, the risk of the procedure is found to exceed the benefit.

Lastly, the user can delay the weaning from cardiopulmonary bypass until the oxygen debt, represented by residual acidosis during reperfusion, is fully paid 110. Weaning from cardiopulmonary bypass in the presence of myocardial acidosis may cause the hemodynamics to deteriorate postoperatively, often prompting the reinstitution of cardiopulmonary bypass. When the heart is subjected to significant ischemia during the period of aortic clamping or reperfusion, a significant amount of time may be needed until the ischemia reverses to normal levels.

In the event that one of the described options, 100 through 110, fails to relieve the ischemic condition, as evidenced by the display of tissue pH levels on the pH monitor, the user can use any of these other described options to attempt to raise tissue pH.

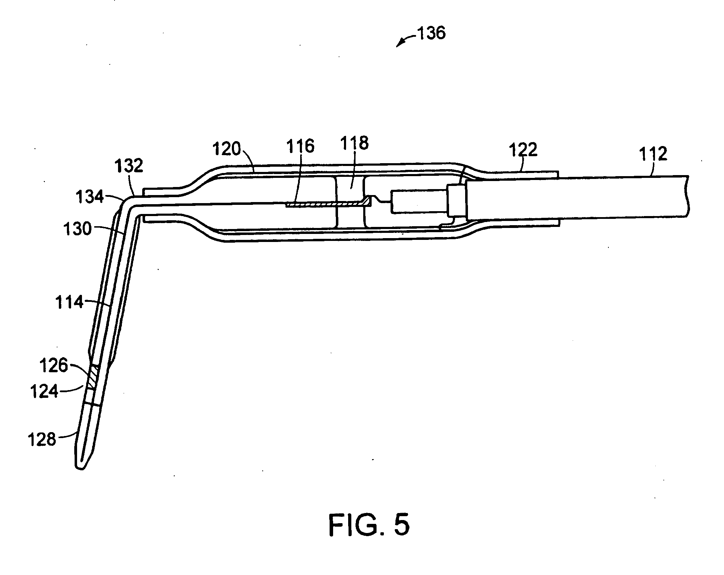

Figure 5 illustrates a preferred embodiment of a pH electrode 136 used to monitor tissue acidosis in accordance with the present invention. The electrode 136 can have a cable 112 connected to a silver wire 114. In a preferred embodiment, the silver wire 114 is an Ag/AgCl (silver/silver chloride) wire. In another preferred embodiment, the cable 112 is connected to the silver wire 114 by a platinum wire 116 passing through a glass seal 118. The cable 112 and wires 114, 116 are encased in a housing 120 which is encased in shrink tubing 122. The electrode 136 has a glass stem 124 which houses the silver wire 114, a thermistor 126, a pH sensor 128, and a gelled electrolyte 130. The electrode 136 can also have a suture groove 132 to allow the electrode 136 to be secured to the site where it is used. The electrode 136 can also have a bendable joint 134 which allows the user to adjust the positioning of the electrode 136 prior to or during use. The glass stem 124 is pointed to allow direct insertion into tissues. In a preferred embodiment, the glass stem 124 is made of lead glass. The electrode can be sterilized by ethylene oxide or gamma irradiation. A pH electrode suitable for use with the invention is available from Vascular Technology Inc., Lowell, Massachusetts and Terumo Corporation of Tokyo, Japan. This particular electrode can be inserted into tissue to a depth of up to 10 mm, has a diameter of 1 mm, and employs a pH sensor in the distal 4 mm of the probe.

Tissue pH is an important clinical measurement. Local acidosis, which can be measured as a distinct drop in pH, has been associated with ischemia. Temperature is preferably measured simultaneously with the pH to allow for the calibration and temperature correction of the tissue pH measurement. Temperature correction of the pH is important, particularly in procedures, such as open-heart surgery, which require significant cooling. The pH electrode uses combination pH/temperature sensors, each of which contains a temperature-sensing element mounted inside the pH-sensing sensor.

Glass pH electrodes are most commonly used to obtain accurate clinical pH measurements. They consist of a hollow glass sensor filled with electrolyte that is in turn in contact with an internal reference wire. Due to the nature of the glass used, an electric potential is developed across the glass. This potential is proportional to the difference between the pH of the analyte solution in contact with the exterior surface of the glass and the essentially constant pH of the internal buffer solution.

In order to make an electrical measurement, a complete electric circuit must be formed. Therefore, a second electrical contact with the analyte solution must be made. This is accomplished through the use of a needle reference electrode. It consists of a silver chloride needle in contact with a constant molarity salt solution. The salt solution is placed in contact with the analyte solution, i.e., the patient's tissue, using a suitable isolation mechanism, in this case through the use of gelled salt solution that has been placed in a flexible tube, the open end of which is placed in contact with the patient.

The Nernst equation predicts that under constant environmental conditions, the output of the glass pH electrode is linear with pH. Therefore, the electrical output of the sensor can be converted to pH by the use of a simple straight-line curve-fit. This requires determining the electrical output of the electrode at two different pH values, from which the slope and offset constants for the straight-line equation can be calculated. The commonly available standards buffers for pH electrode calibration have pH values of 4, 7, and 10. The 4 and 7 pH buffers are preferable for use with a preferred embodiment of the system. The 7-pH buffer is preferable because the electrode's zero-potential point is near pH 7. The 4-buffer is preferable because pH values of the greatest interest lie somewhat below pH 7.

The theoretical sensitivity-the slope-of this type of electrode is 59.16 mV/pH at 25° C. For real electrodes, it tends to be a little less, the value being slightly different from one electrode to another and, for a given electrode, varying over its useful life.

The zero potential point is defined, as that analyte pH value for which the measured output voltage is zero, after correcting for any difference in the salt concentrations of the internal and reference solutions. The zero potential point occurs, therefore, when the analyte pH value is the same as the pH value of the pH sensor's internal buffer. If a measurement is actually made under these conditions, however, a non-zero potential, in general, is measured. This occurs when the Cl concentration that the sensor's internal reference wire is exposed to differs from the concentration that the reference needle is exposed to, or if both reference wires are not made of the same material. In a preferred embodiment of the system, the reference needle is immersed in a saturated KCl gel, while the sensor's internal reference wire is exposed to an 0.87 M concentration of KCl in the internal buffer. This difference results in a measured potential of about + 30 mV at 25° C when the analyte has the same pH value as that of the internal buffers, nominally 6.33 pH at 25° C. Thus, in order to measure the true zero potential point, it is necessary to correct the measured voltage by subtracting 30 mV from it. The 7 pH buffer is used during calibration for zero point calibration is the closest readily available buffer value to 6.33.

Since there is some variation in output from the ideal values as just described, both from sensor to sensor and over extended periods of time for the same sensor, the pH sensors must be calibrated prior to each use. This is accomplished automatically during the calibration procedure by placing the sensors first in the slope buffer (4.00 pH) and then in the zero potential point buffer (7.00 pH). The microprocessor reads the output of the sensors in mV, correcting for the salt differential, determines when the readings are stable and then computes the slope and offset calibration factors for each sensor. Both the slope and zero potential point vary with temperature and are corrected for by the monitor's software.

The pH electrode's combination pH/temperature sensor uses a precision thermistor element to measure temperature. The thermistor is one of the most common temperature measuring devices in use. It consists of a small bead of metallic oxide semiconducting ceramic. The material's electrical resistance varies inversely with temperature in a non-linear manner.

To measure temperature, the thermistor is electrically placed in series with a fixed resistor in the monitor that has precisely known resistance. A voltage is applied across the series combination and the voltage at the junction of the thermistor and resistor is measured. This measured value, in conjunction with the known values of the fixed resistor and of the applied voltage, is used to calculate the resistance of the thermistor. The temperature is then determined by means of a look-up table stored in the microprocessor program. The thermistor sensors used with preferred embodiments of this system are manufactured to a level of precision that makes individual calibration by the user of the system unnecessary.

The pH electrode can be pre-calibrated and packaged such that the tip of the electrode is sealed within a sleeve or a sleeve pocket containing a pH 4.0 buffer. The sleeve pocket can be formed of a plastic material and can have a 3 mm internal diameter. Prior to its insertion in the patient, the sleeve pocket can be removed, the electrode tip wiped dry with, for example, a gauze, and the electrode inserted into a beaker containing a pH 7.0 buffer. The calibration is completed at this point. Packaging the electrode within a pH 4.0 buffer allows the electrode to remain moist through its storage, a factor which is necessary for proper calibration, and reduces the steps required for electrode calibration to a single step. The software in the electrode monitor can be modified to reflect the single step calibration.

The processing unit and monitor, to which the pH electrode, the reference electrode, and thermistor are attached, processes the signals and continually records and displays the following data at 20 second intervals or less: 1) the tissue pH in pH units, 2) the tissue hydrogen ion concentration [H+] in nmoles, 3) the tissue temperature in °C, 4) the pH corrected for 37°C, and 5) the tissue hydrogen ion concentration [H+] calculated as the inverse log of pH. The correction for 37°C is based on a factor of 0.017 pH units /°C which is determined based on a series of measurements. In addition, the monitor allows for the calculation of integrated mean pH, [H+], and temperature over a specific period of time by signaling at the beginning and at the end of the specified period. A slave monitor is attached to the unit and placed in front of the surgeon providing a customized continuous display of the data. The continuous real-time display of the data allows for prompt institution of pH-guided myocardial management to prevent or reverse myocardial tissue acidosis.

Several devices or tools can be used in pH guided myocardial management during cardiac surgery and in the assessment of myocardial viability. The maintenance and distribution of cardioplegic solution to specific myocardial segments during cardiac surgery can be achieved using several different devices and approaches.

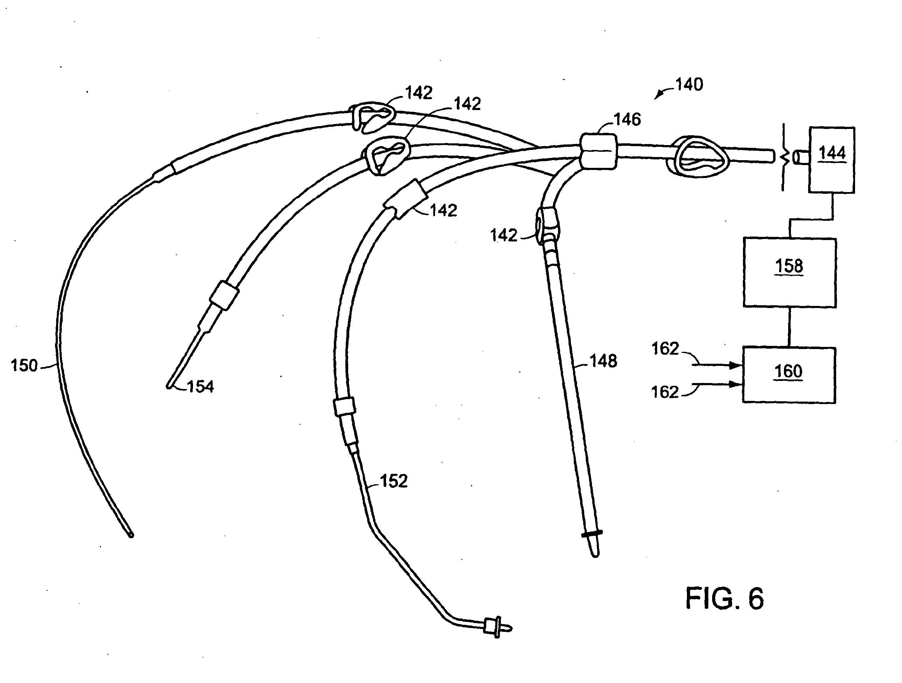

Figure 6 illustrates a "turkey foot" configuration of a cardioplegia delivery system 140 supplied by Medtronic of Grand Rapids, Michigan. The delivery system 140 in conjunction with the electrode can form a myocardial management system. The system 140 can also include a data processing system 160, such as a computer, and a controller 158. The data processing system 160 can be programmed to receive measured data 162, such as the status of the patient and changes in system status. The data processing system 160 can be attached to a fluid source of fluid delivery system 144. The data processing system 160 can also be attached to the fluid source through the controller 158. The controller 158 can operate the fluid delivery system. The controller 158 can control the flow rate of a preservation fluid or cardioplegia fluid delivered to a surgical site. The controller 158 can also control the temperature of a preservation solution and a delivery site of a preservation solution. The system 140 has a plurality of controls 142 which can be used to adjust and selectively administer the amount of cardioplegia solution delivered from a source 144 to various cardiac attachment sites. The system 140 can include an occluder or valve 146 which controls the flow of the cardioplegic solution.

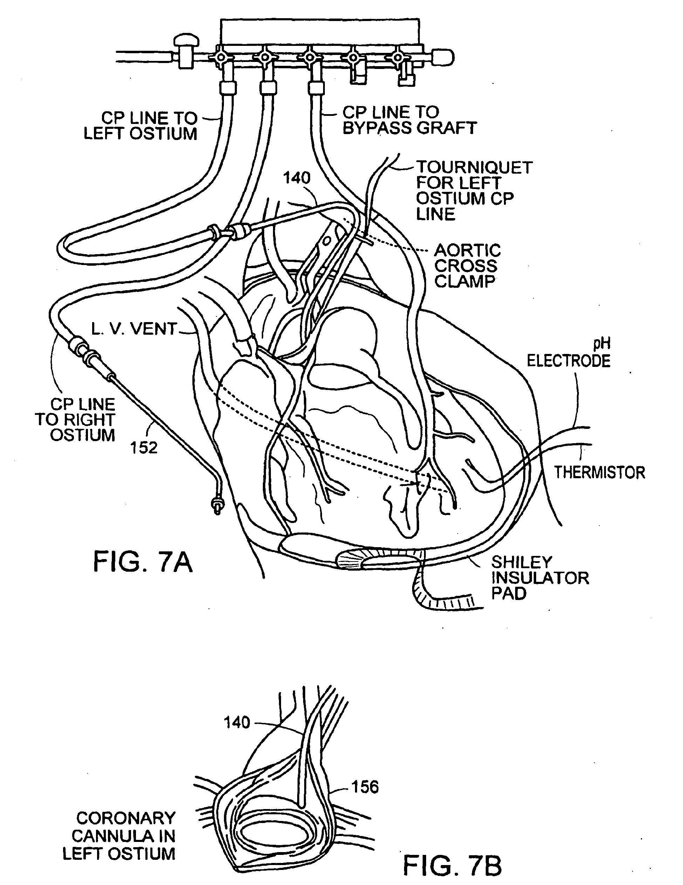

The system 140 includes several delivery devices attached between the cardioplegia source 144 and various cardiac sites. These devices allow the delivery of cardioplegic solution to their respective cardiac sites. One device is a cannula 148 (Sarns Inc., Ann Arbor, Michigan) which can be inserted in the aortic root. Another device is a Spencer cannula 150 (Research Medical, Inc., Midvale, Utah) which can be inserted within the orifice 156 of the left main coronary artery. This insertion into the orifice 156 is shown in Figures 7A and 7B. Another device is a malleable metallic catheter 152 (Medtronic, Grand Rapids, Michigan) which can be inserted within the orifice of the right main coronary artery. The catheter 152 is also shown in Figure 7A in an uninserted state. Another device is a 14 gauge beaded needle (Randall Faichney Corp., Avon, Massachusetts) which can be attached to the proximal end of a - saphenous vein graft for the delivery of cardioplegia. The attachment to the vein graft is also shown in Figure 7A.

Blocking the orifice of the left main coronary ostium with a spherical catheter such as a Spencer cannula 150 (Research Medical, Inc., Midvale Utah) or balloon tipped catheter such as a #3F Fogerty Catheter (Ideas For Medicine, St. Petersburg, Florida), while providing cardioplegia through other sites of 140, can also be used to redistribute cardioplegia solution during cardiac surgery. Also, applying temporary occlusive pressure to a coronary artery proximal to the site of insertion of a new vein graft while perfusing a cardioplegic solution through the proximal end of the graft can also be used to re-direct cardioplegic fluid during cardiac surgery. Occlusive pressure can be maintained with a gauze "peanut" at the tip of a Kelly clamp (Allegiance Healthcare Corp., McGaw Park, Illinois).

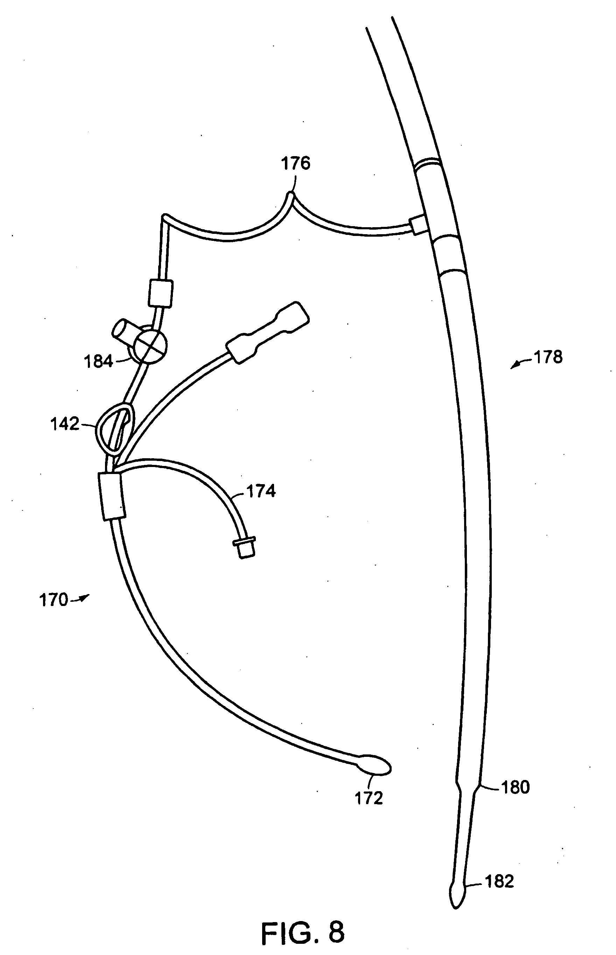

A Guntrie balloon tipped cannula (Medtronic, Grand Rapids, Michigan) can also be attached to the system 140 and inserted in the coronary sinus for selective administration of cardioplegia in a retrograde manner. The cannula 170 is illustrated in Figure 8. In this figure, it is illustrated as being attached through tubing 176 to the venous cannual 178. This allows manipulating the pressure in the coronary sinus to improve cardioplegia delivery to the tissues as part of pH-guided myocardial management. The pressure can be manipulated by inflating a coronary sinus balloon 172 with the fluid orifice of the coronary sinus catheter closed, and delivering the cardioplegia antegrade. The 1mm tubing 176 connecting 170 to 178 creates back pressure which improves delivery without interfering with adequate antegrade cardioplegia flows. The opening or closing of the fluid orifice of the coronary sinus catheter 170 can be controlled by a valve 184. The venous cannula 178 is normally inserted in the course of cardiopulmonary bypass with its tip 182 in the inferior vena cava and its more proximal orifice 180 in the right atrium.

Changing the tissue temperature by manipulating the temperature of the cardioplegic solution using a water heater/cooler, such as that manufactured by Sarns, Ann Arbor, Michigan, can aid in managing myocardial pH during cardiac surgery. Also, changing the perfusion pressure of the cardioplegic solution by changing the rate of cardioplegia flow using a cardioplegia system such as an HE30 Gold cardioplegia system (Baxter Corporation, Irvine, California) can aid in managing myocardial pH during cardiac surgery.

Tools can also be used for the assessment of myocardial viability and the determination of the physiologic significance of coronary stenosis. The tools can be used in either an operating room or a cardiac catheterization lab.

In the operating room, pacing wires, for example, manufactured by Ethicon of Somerville, New Jersey can be placed over the right atrium and connected to an external pacemaker manufactured by Medtronic of Grand Rapids, Michigan. A pH electrode can also be inserted into the myocardium. A fall in myocardial pH in response to 5 minutes of rapid atrial pacing can indicate tissue ischemia and also can indicate that the myocardial segment in which the electrode is placed is viable.

In the cardiac catheterization laboratory, the pH electrode can be mounted at the tip of a long 0.014 gauge wire and inserted through a regular 6 french cardiac catheterization catheter such as that manufactured by Cordis of Miami, Florida. The catheter tip can be positioned perpendicularly against the ventricular wall of the segment subtended by the coronary artery being investigated and the pH electrode pushed to penetrate into the subendocardium. Preferably, the electrode is pushed to penetrate approximately 5 mm into the subendo cardium. Pacing is achieved via a pacing wire advanced into the right ventricle and attached to an external pacemaker (Medtronic, Grand Rapids, Michigan). Again, a fall in myocardial pH in response to 5 minutes of rapid arterial pacing can indicate tissue ischemia.

While the pH electrodes and monitoring system have been described for use in determining the ischemia of cardiac tissue, the pH system and methods can be used in other types of tissue as well. The pH system can be used to monitor rejection in organ transplantation, to assess mesenteric ischemia, to monitor and assess brain blood flow and to monitor flaps in plastic surgery.

The pH electrode can be used to monitor the acid base state and the adequacy of the preservation of the donor heart during excision, transportation, and inserting in the patient in the course of cardiac transplantation.

The pH electrode can be used to monitor the kidney in the course of and following kidney transplantation. The pH electrode can be used in the monitoring of tissue perfusion to the kidney in the course of major surgery and, in particular, during kidney transplantation. The electrode is readily implantable in the kidney in a manner similar to the heart, and a tissue pH level of 7.2 and above indicates adequate tissue perfusion. Damage to the kidney, particularly during excision of the kidney for the purpose of donor related cardiac transplantation, can be detected and avoided, thus insuring a better outcome of the donor related kidney transplantation. Preservation of the kidney during transport prior to transplantation can also be insured by monitoring and maintaining the pH at normal levels. This can be achieved with constant perfusion of the kidney with blood in a specially designed apparatus for organ perfusion.

Following kidney transplantation, keeping the electrode in the kidney throughout the immediate 48 hours post-operatively can allow for monitoring initial ischemia and can allow for reversing of this ischemia with operative interventions. Ischemia during this period can herald a significant bad outcome. Assessment of the transplanted kidney, function and detection of its rejection can also be performed by placing the electrode on a catheter and passing it retrograde into the calyx of the kidney. Puncturing the calyx of the kidney along with the kidney parenchyma, similar to what was described above for the heart, can indicate impending or actual rejection and, as such, is indicative of adverse outcome. Early detection of acidosis can prompt major treatment of rejection, and thus can improve the outcome of kidney transplantation.

Each electrode can be used also for the assessment of the adequacy of the revascularization of the kidney in the course of renal artery revascularization. The efficacy of the revascularization of a critically stenod renal artery can be determined intra-operatively in a manner similar to the efficacy of the revascularization of the coronary arteries. Failure to reverse acidosis with revascularization prompts additional intra-operative measures to reverse the acidosis, and hence, avoids adverse outcome of revascularization. As in the heart, failure to reverse the acidosis with revascularization is indicative of the inadequacy of the revascularization process and provides a guide for additional intra-operative management to improve the situation and improve the outcome of the revascularization.

The pH electrode can also be used to monitor the liver during and following liver transplantation. The pH electrode can be inserted into the liver to provide important data similar to that of the kidney, described hereinbefore. The description of the use of the electrode in the kidney is applicable to the liver in terms of the use of the pH electrode in monitoring the intra-operative course, identifying early rejection, and instituting measures to reverse the rejection process.

The electrode can also be used in monitoring the periphery during and following major peripheral revascularization and in critical care. Insertion of the electrode in the subcutaneous tissue of the periphery provides information on the adequacy of tissue perfusion. Acidosis measured at these sites, primarily in the subcutaneous tissue of the distal half of the lower extremity, can indicate peripheral arterial obstruction or an inadequate cardiac output, and can prompt the institution of measures to improve cardiac output or tissue perfusion. These measures can include direct revascularization surgery or pharmacologic manipulations and/or insertion of an intra-aortic balloon such as, for example, manufactured by Arrow International of Reading, Pennsylvania in the descending aorta, for example. Currently, only measures of central hemodynamics are used to assess and treat low cardiac output syndrome. Measuring the pH in the periphery provides a more superior alternative because it provides a true measure of tissue perfusion which is the ultimate goal in the maintenance of an "adequate" cardiac output.

The pH electrode can also be used within the muscle and subcutaneous tissue of flaps in plastic surgery. It has been demonstrated that tissue acidosis with the pH electrode indicates compromised viability of skin and subcutaneous flaps. The electrode is placed post-operatively within the edge of the flap and the pH is monitored up to three or four days post-operatively. A fall in pH prompts an intra-operative intervention and a revision of the flap to prevent its subsequent failure.

The pH electrode can also be used in the colon in the assessment and treatment of intestinal ischemia. To assess and reverse intestinal ischemia, the pH electrode can be placed on a wire in a manner similar to that described for the heart during cardiac catheterization hereinbefore. The pH electrode-tipped wire can be inserted through a colonscope, such as that manufactured by Olympus Medical of Seattle, Washington, during regular colonoscopy into the distal ileum. Intra-luminal pH in the ilium is a reliable measure of the adequacy of the perfusion. Intra-luminal acidosis in the ilium indicates intestinal ischemia, and can prompt maneuvers to either reverse the ischemia or to prevent its adverse outcome. Knowledge of intra-luminal pH in the ilium allows the initiation of operative interventions, such as exploration of the abdomen with the possible resection of intestine, for example, as well as pharmacologic interventions to improve cardiac output and tissue perfusion.

The pH electrode can be used in other organs. In addition to the organs mentioned above, tissue acidosis can be measured, manipulated, and reversed by inserting the pH electrode, attached to the pH monitoring system, in organs such as the brain, the bladder, the diaphragm, and the small intestine.

The myocardial pH monitoring system in accordance with a preferred embodiment provides a sensitive online tool to assess both the adequacy of myocardial protection during the interruption of coronary blood flow and the adequacy of revascularization following the construction of aortocoronary bypass grafts. The pH-guided myocardial management prevents and reverses myocardial tissue acidosis during all phases of the cardiac surgery operation. Myocardial tissue acidosis has been demonstrated to be a reliable indicator of the magnitude of myocardial ischemia. Regional myocardial acidosis is also frequently encountered, in an unpredictable manner, in the course of cardiac surgery in humans. Recent studies have shown a direct relationship between the magnitude of regional myocardial acidosis encountered intraoperatively and the incidence of adverse postoperative patient outcomes. Severe intraoperative myocardial acidosis has also been shown to decrease long-term survival following cardiac surgery. These observations gain added significance in light of recent experimental studies that have implicated acidosis as a primary trigger of myocyte apoptosis under conditions of ischemia and reperfusion, and as a possible cause of late heart failure.

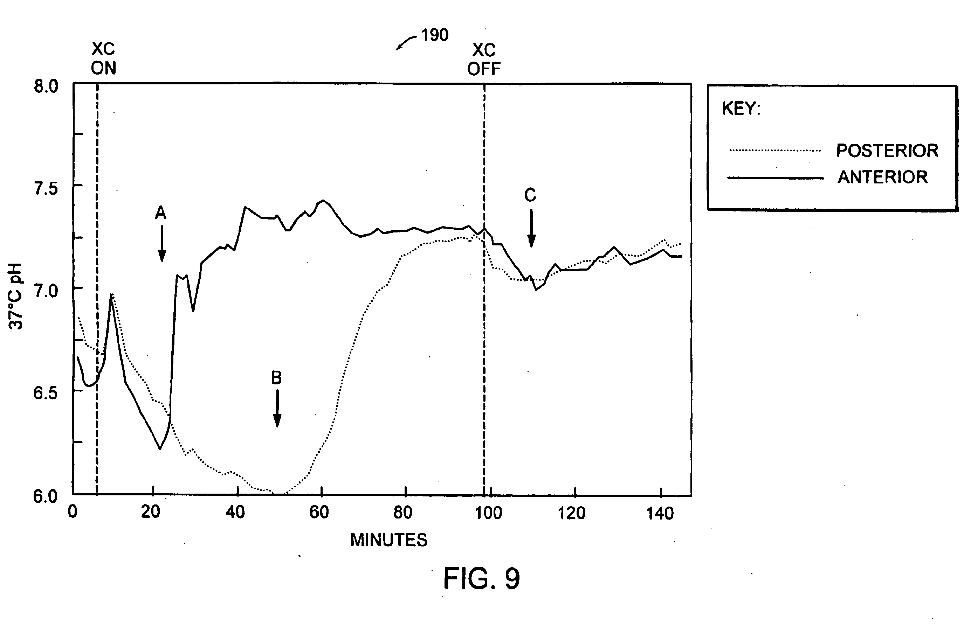

Figure 9 graphically illustrates the pH monitored at the anterior and posterior wall of the left ventricle in accordance with the present invention. Myocardial pH (correct to 37° C) was measured and recorded in both the anterior and posterior walls of the left ventricle. The electrodes were inserted at 0 minute and the aorta was cross-clamped (XC) 5 minutes later. An initial bolus of warm blood cardioplegia given through the aortic root resulted in an increase in the pH in both walls. Cardioplegia delivery is then interrupted, and a segment of saphenous vein is sutured to the left anterior descending coronary artery (LAD). During this period the pH fell to 6.2 in the anterior wall and 6.4 in the posterior wall.

Once the anastomosis to the LAD was completed, continuous blood cardioplegia was delivered through the proximal end of the graft (Arrow A) and maintained throughout the rest of the procedure. It resulted in a prompt rise in the anterior wall pH, indicating adequate cardioplegia delivery to the anterior wall and technical adequacy of the graft anastomosis. A few minutes later, as the aorta was opened and the aortic value replacement was started, continuous warm blood cardioplegia was given retrograde through the coronary sinus in addition to the LAD graft. However, over the next 30 minutes, pH in the posterior wall continued to fall to low acidic levels despite the simultaneous administration of cardioplegia through the LAD graft and the coronary sinus, and despite the simultaneous administration of cardioplegia through the LAD graft and the coronary sinus, and despite other maneuvers which included delivering the cardioplegic solution through the coronary sinus only, doubling its flow rate through both sites, and cooling it to 15 C. Based on an excessive return of blood which was observed coming through the orifice of the left main coronary artery, it was suspected that a steal phenomenon might be occurring. At the point in time indicated by Arrow B, a Spencer canula was placed in the orifice of the left main coronary artery and clamped, thus driving the cardioplegic solution through the LAD graft distally instead of proximally. This maneuver effected a prompt and dramatic rise in the posterior wall pH, confirming the previously suspected steal phenomenon.

Throughout the last 30 minute-period of crossclamping, delivery of the cardioplegic solution through the LAD graft alone was enough to maintain normal pH in both the anterior and posterior walls. Retrograde cardioplegia was discontinued during this period because it was ineffective in protecting the posterior wall. Following release of the aortic clamp (XC off), the patient defibrillated spontaneously and weaned from cardiopulmonary bypass without inotropic support in his postoperative course. After completing the proximal anastomosis, flow through the LAD graft was restored (Arrow C) causing the pH to normalize in both walls (normal myocardial pH range is 7.1-7.3). The integrated mean pH during the period of aortic clamping (which has been shown to be predictive of outcome) was 7.12 in the anterior wall and 6.60 in the post wall. Had myocardial pH not been monitored, the acidosis in the post wall would have been much more severe, and the likelihood of a compromised patient outcomes would have been greatly increased.

Acidosis can prematurely trigger and accelerate cell apoptosis, or programmed cell death. In the heart, apoptosis may manifest in late adverse outcomes, mainly progressive heart failure. During the course of open heart surgery, moderate to severe acidosis is encountered, at least in one segment of the left ventricle, in more than 50% of the patients. The prevention of the onset of myocardial tissue acidosis by pH-guided myocardial management in the course of open heart surgery reduces or eliminates the potential of triggering apoptosis, and hence reduces or eliminates the potential of late adverse postoperative outcomes.

In accordance with another aspect of a preferred embodiment of the present system includes the recognition that there are many indications that cardiac surgery is a major potential cause of patient injury, as discussed hereinbelow. First, despite relatively low 30-day mortality rates following cardiac surgery in general, the mortality and morbidity rates remain unacceptably high in relatively large subsets of patients undergoing cardiac surgery. Currently, nearly one third of patients are likely to die or to sustain a myocardial infarction after coronary artery bypass surgery in certain high risk patient populations. These relatively high postoperative morbidity and mortality rates indicate that total patient safety in these patient groups remains elusive even today.

Second, there are still many patients who are denied cardiac surgery because of what is perceived as an unacceptably high risk of operative mortality. This indicates that surgeons are worried that these patients might incur serious injury during or after the operation.

Third, a low 30-day postoperative mortality rate is not as good an indicator of the safety of cardiac surgery. Cardiac surgery continues to be accompanied with increased 30-day morbidity, poor long-term outcomes, and added costs, even when the 30-day mortality is low. It is not unusual for a cardiologist to encounter late postoperative heart failure in a patient whose hospital course after cardiac surgery was totally uneventful. Acidosis has been implicated as a primary trigger of apoptosis, as well as the demonstration that cardiac apoptosis can lead to heart failure, suggest that apoptotic changes might be triggered in the course of a cardiac operation, thus effecting an injurious cascade of adverse clinical events that become manifest late in the postoperative course.

Fourth, 30-day postoperative mortality rates are not invariably low at all institutions. When properly adjusted for preoperative risk, the 30-day mortality rate is a reliable comparative measure of the quality of surgical care among various institutions. This rate has been shown to variate by a factor of four between various institutions. These considerations indicate that patient safety is being jeopardized in cardiac surgery. An understanding of the potential sources of patient injury in cardiac surgery is paramount to improving safety and, hence, the long-term outcomes of cardiac surgical patients.

One important source of patient injury is the interruption of blood supply to the heart as discussed hereinbefore. In almost every cardiac operation, the heart is temporarily deprived of its blood supply, either regionally or globally. Considering that progressive pathologic ischemic changes start to occur in the myocardium within minutes after the interruption of its blood flow, a time-dependent hazard for myocardial injury is present in every patient undergoing cardiac surgery. The compendium of methods that have been described hereinbefore as myocardial protection techniques are aimed primarily at prevention of this type of injury, although these techniques are limited in their ability to achieve complete protection of the heart in all patients undergoing cardiac surgery. The time-dependence of this type of injury limits the protective efficacy of current myocardial protection techniques, particularly in complex operations requiring prolonged periods of aortic clamping. Both the duration of the period of aortic clamping and the duration of cardiopulmonary bypass have been consistently shown to be the main determinants of postoperative outcomes in all studies that have attempted to identify the determinants of outcomes of cardiac surgery.

The on-line measurement of myocardial tissue pH and its potential to guide interventions that prevents the onset of, or reverse, myocardial tissue acidosis in real time is an important recognition of the present invention. Late postoperative outcomes and long-term survival are reflective of the adequacy of intraoperative myocardial management.

Two important variables are determinants of long-term outcome and survival after cardiac surgery: the degree of myocardial tissue acidosis during the period of aortic clamping, and the early postoperative left ventricular ejection fraction. In a study with an average follow-up of 10 years after complex cardiac surgery, a direct relationship between the lowest mean myocardial pH recorded both during and after the period of aortic clamping, and long-term patient survival is observed from a series of procedures. Patients who experienced acidosis during various period in the course of an operation have decreased survival compared with those who did not. Because myocardial acidosis is a predicate to preferred embodiments of the present invention, and is reflective of both myocardial ischemia and poor myocardial protection, the adequacy of intraoperative myocardial protection to long-term outcome is determined in a preferred embodiment and indicates that prevention of intraoperative acidosis improves long-term survival after cardiac surgery.

As recognized by the preferred embodiments of the present invention, one of the most sensitive markers of inadequate preservation of the myocardium and of the onset of myocardial ischemia is the development of myocardial tissue acidosis. The myocardium has the highest fractional oxygen extraction capability of any organ in the body, at a rate of nearly 70%. Myocardial metabolism is almost entirely aerobic. Under conditions of normal metabolism and adequate myocardial perfusion, the production and washout of hydrogen ions are in equilibrium, resulting in a normal tissue acid-base balance. Glycolysis, glycogenolysis, hydrolysis of adenosine triphosphate (ATP), hydrolysis of triglycerides, and the synthesis of triglycerides from palmitate are all sources of hydrogen ion production in the myocardial cell. Under global ischemic conditions, when the myocardium is almost totally deprived of its oxygen supply, the major source of ATP is anaerobic glycolysis. In this state, there is an intracellular decrease in high-energy phosphates and an increase in inorganic phosphate. With increasing duration of ischemia, glycolysis is inhibited by increasing levels of lactate and hydrogen ions. Anaerobic glycolysis thus ceases after a period of 90 minutes. The accumulation of hydrogen ions in this situation is due to an increased production from anaerobic glycolysis, glycogenolysis, and ATP hydrolysis, combined with decreased washout due to either diminution or cessation of blood flow. During regional ischemia, oxygen is available to the myocyte in reduced concentrations. When the supply for oxygen fails to meet the tissue demand, the intracellular production of hydrogen ions increases. The hydrogen ion then accumulates in the myocardial tissue if its rate of production exceeds the rate of its washout by the regional myocardial blood flow. It is important to underscore that the accumulation of the hydrogen ions, under conditions of both global and regional myocardial ischemia, is dependent on both its rate of production and its rate of washout.

Interruption of the coronary flow to a segment of the myocardium results in a rapid accumulation of both tissue hydrogen ion and CO₂ in that segment. Tissue acidosis results in increased hydrogen ion and CO₂ production through the carbonic anhydrase reaction. A peak concentration of these metabolites is reached 30 to 45 minutes after the interruption of flow. The maximal rate of accumulation of these metabolites and the peak tissue concentration reached are proportional to the magnitude of the ischemic insult inflicted by the interruption of the blood flow. After reaching a peak, the concentrations of both hydrogen ions and CO₂ gradually decline. This decline is indicative of progressive ischemic cellular dysfunction, and indicates that the ability of the cell to produce hydrogen ion and CO₂ is an index of its viability. Metabolically dysfunctional and dead myocardial tissues do not exhibit a rise in hydrogen ions and CO₂ in response to an interruption of blood supply to these tissues.

Depression of myocardial contractility and function is known to occur in the setting of acidosis. Hydrogen ion accumulation depresses myocardial contractility through direct actions on the myocyte and through interactions with intracellular calcium. Myocardial Pco₂ has been shown to decrease contractility as well. Under conditions of global ischemia, the magnitude of tissue acidosis incurred throughout the period of aortic clamping, and the rate of rise in tissue [H⁺] throughout the first 10 minutes of reperfusion are important predictors of postischemic cardiac dysfunction. Reducing the magnitude of tissue acidosis during the periods of global ischemia and reperfusion reduces postischemic cardiac dysfunction.

Myocardial tissue acidosis can be quantified by the measurement of tissue Pco₂ or hydrogen ion [H⁺] concentration. Mass spectrometry has been adapted to the online measurement of myocardial tissue Pco₂. Although reliable data can be obtained in the experimental laboratory with this technique, it cannot be brought to the operating room because of inherent limitations such as relatively long stabilization and response times.

The pH and hydrogen ion sensing system in a preferred embodiment is comprised of a sensing electrode, a reference electrode, and a monitor that provides continuous online outputs with recording capabilities. As described hereinbefore with respect to Figure 5, the electrode is a plunge-type probe that is approximately 10 mm in length and 1 mm in diameter, with a lead glass, silver/silver-chloride sensing surface. The electrode also contains a thermistor for the simultaneous measurement of myocardial temperature. The reference electrode is kept at room temperature and connected to the subcutaneous tissues with a salt bridge. The computerized monitoring system, after calibration of the electrodes, records and displays in real-time the myocardial temperature, pH, and [H⁺]. Myocardial pH and [H⁺] readings are corrected for the effect of temperature and corrected to 37°C. The pH electrode has a 95% response time of 5-15 seconds in a preferred embodiment. It is stable, with a drift of less than 0.1 pH unit over a 6-hour period.

Under ischemic conditions, myocardial pH measurements correlate with other established markers of ischemia including myocardial tissue Pco₂, local intramural ST-segment changes, regional myocardial blood flow, contractile function, and intracellular high-energy phosphate stores.

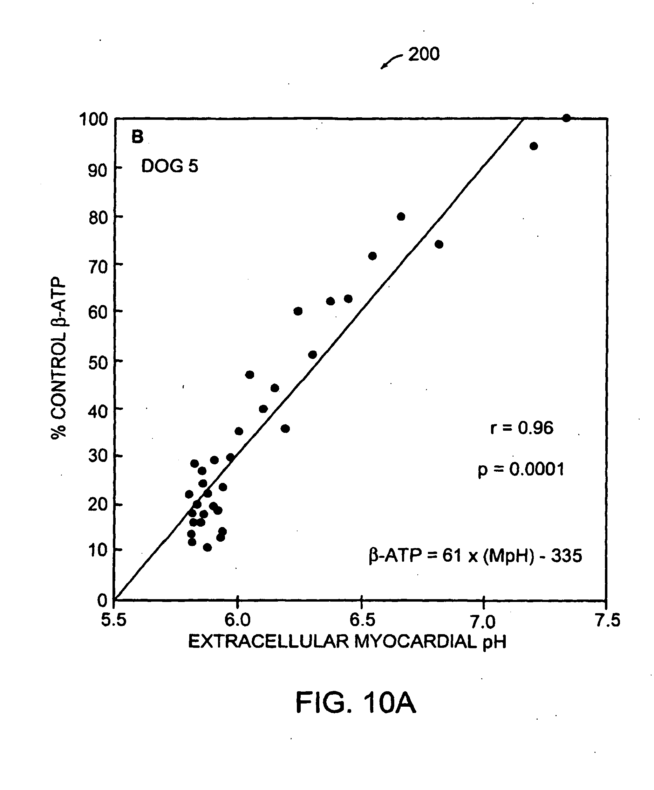

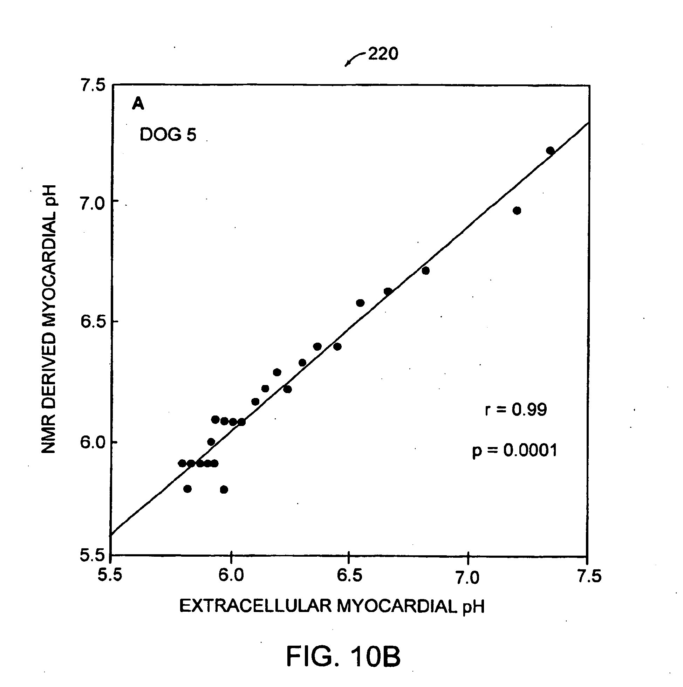

The electrode in accordance with a preferred embodiment is sensitive in depicting regional ischemic events in the conscious chronic canine model and in measuring global ischemic changes in canine subjects placed on cardiopulmonary bypass. Simultaneous measurements made with the electrode and with nuclear magnetic resonance (NMR) spectroscopy in canine hearts subjected to regional ischemia show a high level of correlation between electrode-derived pH of the present invention and NMR-derived pH. Electrode-derived pH in these procedures also correlate well with changes in myocardial tissue ATP, as shown in Figures 10A and 10B.

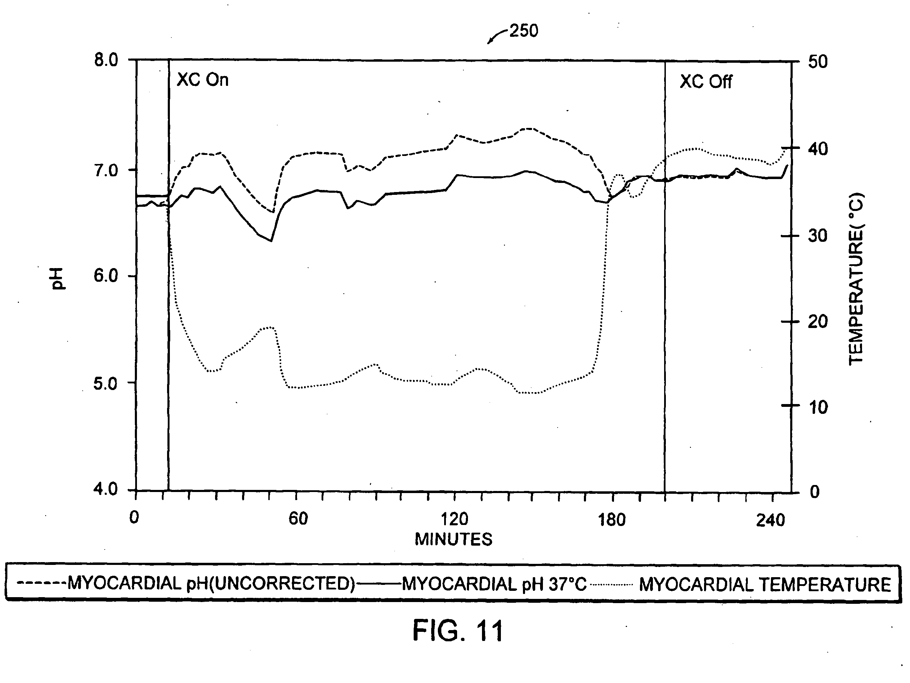

There is a direct relationship between the temperature of blood or tissues and the pH, which is independent of the production and washout of acid. Hypothermia alone results in a rise in tissue pH. It has been demonstrated in a variety of animal species that for every degree celsius of change in tissue temperature, there is a corresponding change of 0.017 units in pH. Failure of the pH to rise in the presence of hypothermia is thus indicative of relative acidosis. This correction factor and its determinants are built into the pH monitor of a preferred embodiment of the present invention. In the face of wide fluxes in myocardial temperature such as one encounters during the delivery of cold cardioplegia, the pH monitor displays the pH or the [H⁺] continuously corrected for 37°C. This allows the surgeon to control for the effects of temperature on pH and to determine the changes that are due solely to acid-base metabolism shown in Figure 11.

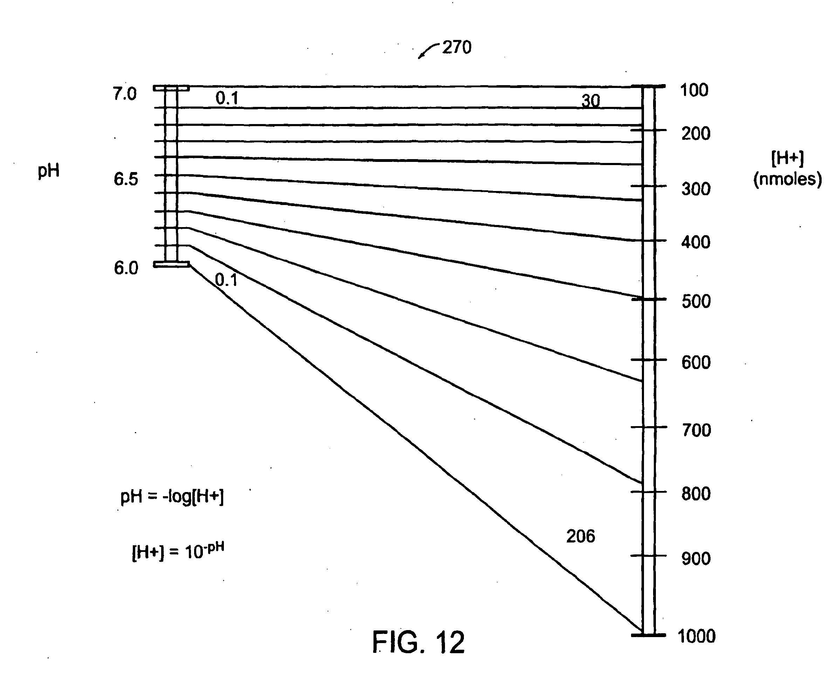

The relationship of pH to hydrogen ion is logarithmic: pH = log [H⁺]. Therefore, changes of equal magnitude along the pH scale do not reflect equal corresponding changes in hydrogen ion concentrations. As shown in Figure 12, which illustrates the relationship between pH in units and corresponding [H⁺] in nanomoles a drop in pH from 7.0 to 6.9 reflects an increase of 30 nmol/L in [H⁺]. The same drop of 0.1 pH units from 6.1 to 6.0 reflects an increase of 206 nmol/L in [H⁺]. When evaluating changes in myocardial pH during ischemia, it should be noted that a progressive fall in myocardial pH is reflective of exponentially higher amounts of acid being produced.

The integrated mean pH during the period of aortic clamping is a determinant of the adequacy of myocardial protection. Low integrated mean pH levels during this period are directly related to poor myocardial protection. Myocardial protection is assessed by a clinical score, which is devised on the basis of the intraoperative and postoperative need for inotropic support, creatine kinase isoenzyme levels, electrocardiographic changes, and results of radionuclide ventriculography. Deleterious clinical effects of acidosis associated with prolonged ischemia are observed despite recorded low myocardial temperatures.

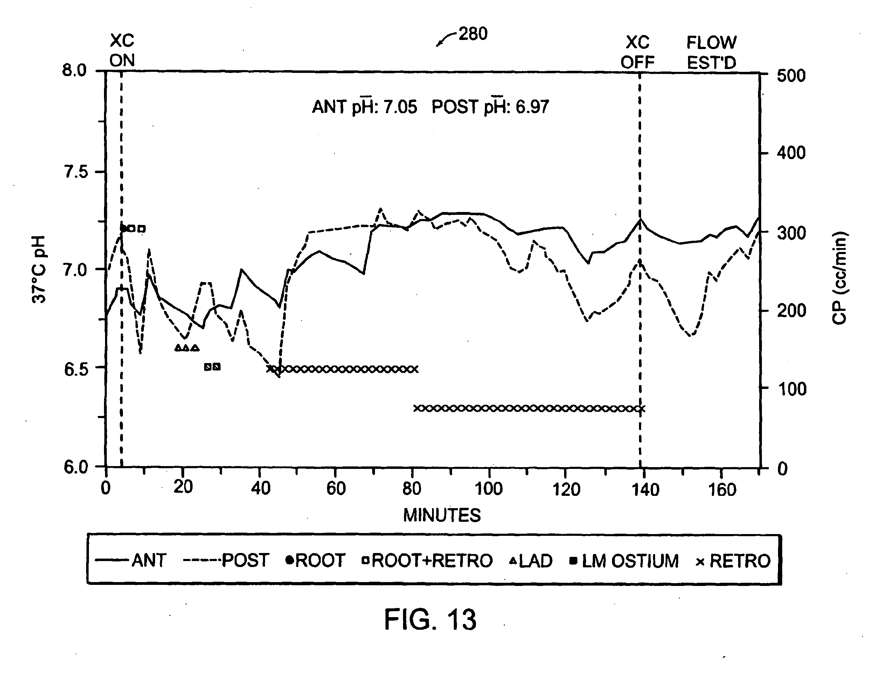

Figure 13 shows the myocardial pH tracings, corrected for 37°C, obtained online in the course of an aortic valve replacement and CABG to the left anterior descending coronary artery (LAD) in a 51-year-old man. The figure provides a record of the blood cardioplegia delivery technique by showing the site, duration, and rate of delivery of the blood cardioplegia solution. The figure also provides an example of adequate myocardial protection in both the anterior and the posterior walls. The cross-clamp period shown between two broken vertical lines is 2 hours 20 minutes, during which myocardial temperature is kept at approximately 20°C. Temperature-corrected myocardial pH is labeled on the ordinate as 37°C pH, i.e., pH is expressed as if myocardial temperature had remained constant at 37°C throughout duration of operation. Sites of cardioplegia delivery are indicated by symbols plotted according to the duration of delivery shown on abscissa and rate of cardioplegia delivery shown on ordinate. Except for period during which the distal saphenous vein graft is being sutured to LAD, blood cardioplegia is continuously delivered, initially through the graft to LAD and subsequently retrograde through the coronary sinus. Mean integrated pH during total period of aortic clamping is 7.05 in anterior wall and 6.97 in posterior wall, indicating adequate myocardial protection achieved mainly with retrograde delivery. Adequate metabolic protection is commensurate with mean pH of 6.8 or greater during aortic clamping. After release of clamp, pH fell in posterior wall but was reversed with institution of blood flow through the graft after completion of proximal anastomosis. This patient experienced spontaneous defibrillation after nearly 2.5 hours of aortic clamping and did not require inotropic support either at weaning or any other time postoperatively. (Ant = anterior wall; CP = cardioplegia, Est'd = established through the graft; LAD = cardioplegia delivery through proximal end of LAD graft; LM Ostium = cardioplegia delivery through ostium of left main coronary artery; Post = posterior wall; Root = cardioplegia delivery through aortic root; Retro = retrograde delivery of cardioplegia through coronary sinus; XC = cross-clamp.)

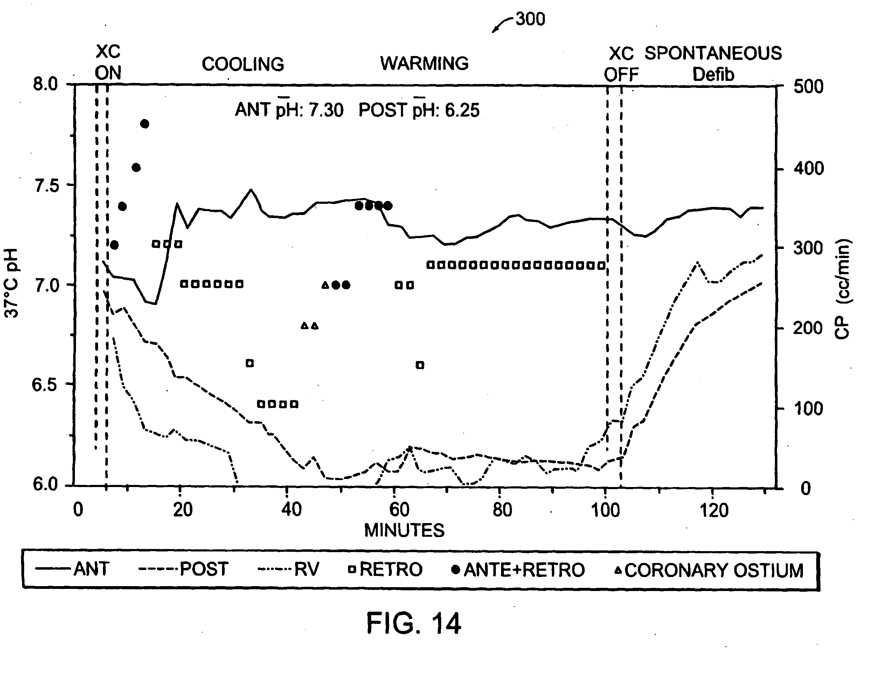

In contrast, Figure 14 exemplifies a clinical situation in which the anterior wall is adequately protected but the posterior wall is not. Temperature-corrected pH reached low of 6.0 in posterior left ventricular (LV) wall and 5.8 in right ventricular (RV) wall. This marked discrepancy between anterior and posterior wall pH occurred in face of continuous delivery of blood cardioplegia at high rates, mostly through the coronary sinus and the aortic root. At the time pH guided maneuvers that would reverse acidosis were not known and cardioplegia were being delivered both antegrade and retograde. Integrated mean pH during period of aortic clamping was 7.30 in anterior LV wall, 6.25 in posterior LV wall, and 6.05 in anterior RV wall, indicating poor protection of posterior LV wall and anterior RV wall. The heart was defibrillated three times and the patient required significant inotropic support to wean from cardiopulmonary bypass; he continued to require inotropic support for 24 hours postoperatively. Note that the symbols ▲ = coronary ostium cardioplegia; □ = retrograde cardioplegia; ● = antegrade and retrograde cardioplegia. The clinical experience in more than 700 patients has shown that patients in whom the integrated mean pH in both the anterior and posterior walls is kept at 6.8 or greater are most likely to wean from cardiopulmonary bypass without significant inotropic support, even when the period of aortic clamping exceeded 3 hours.

Over the past two decades, new strategies for myocardial protection have been developed. These strategies have revolved around the composition, temperature, and route of administration of cardioplegia to the myocardium. Myocardial pH monitoring has provided a new tool for the clinical evaluation of the efficacy of these various modalities. In studies comparing crystalloid with blood cardioplegic solutions, lower levels of tissue acidosis are recorded during the periods of aortic clamping and reperfusion with continuous cold blood cardioplegia. This correlates with lower inotropic and mechanical support postoperatively, and establishes the superiority of continuous cold blood over crystalloid cardioplegia in the prevention of intraoperative myocardial acidosis. Another analysis examined the determinants of regional acidosis in 140 myocardial segments in the course of complex cardiac surgery. It underscored the lack of delivery of the cardioplegic solution to a specific myocardial segment as the only significant determinant of the onset of myocardial acidosis in that segment. Neither the volume of the cardioplegic solution administered, nor its method of administration, nor its temperature influenced the onset of myocardial acidosis.

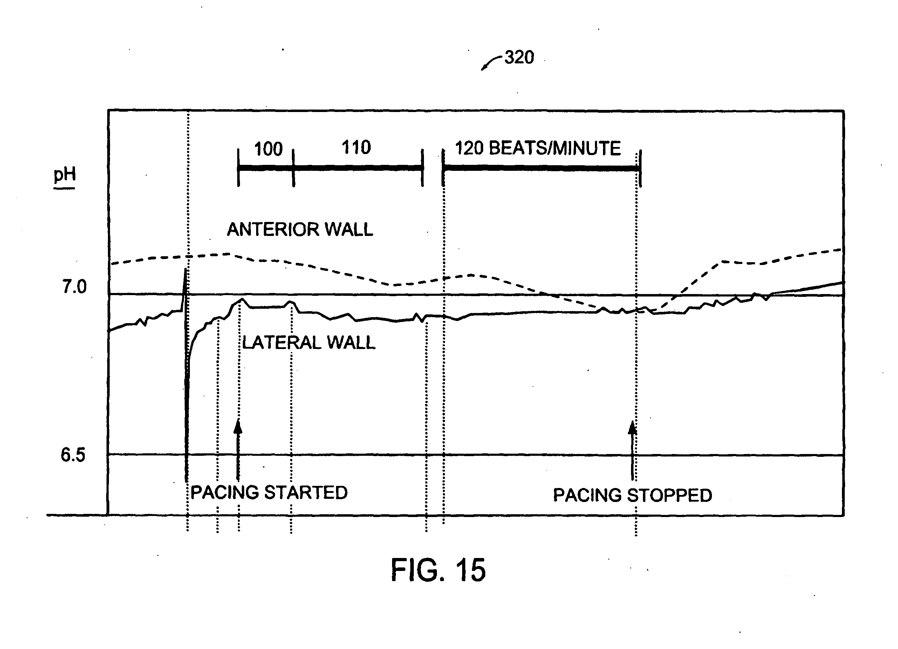

Figure 15 illustrates the response of anterior and posterolateral wall pH to rapid atrial pacing. Bars on top show pacing rate. Pacing for 5 minutes at 120 beats/minute resulted in fall in anterior wall pH. pH in posterolateral wall remained unchanged. The comparative response indicate ischemia in the anterior wall and not the posterior wall. Based on this response, the coronary artery supply to the anterior wall was not revascularized.

The pH electrode of a preferred embodiment has also been used to assess the physiologic significance of a critical coronary artery stenosis. Although coronary angiography provides an adequate assessment of the anatomic extent of coronary artery disease in general, there are situations in which the physiologic significance of questionable angiographic lesions needs to be ascertained. Situations are also encountered intraoperatively in which angiography may not be available or feasible. Under these conditions subjecting the heart to increased demand through atrial pacing, while continuously measuring myocardial pH, creates a "stress test" in which myocardial pH falls if the coronary obstruction is physiologically significant. A pacing-induced fall in myocardial pH of 0.01 units or more, which is reversed after adequate revascularization, is indicative of a physiologically significant stenosis, even if this stenosis is not readily confirmed by angiography. This modality of the present invention assists the surgeon in identifying, in the context of the operation, physiologically significant lesions that should be bypassed; it also provides a tool for detecting instances in which inadequate revascularization has occurred. Figure 15 shows the pH in the anterior and posterolateral walls of the left ventricle in a man who presented with malignant ventricular arrhythmias. The response to pacing described above helped to uncover unsuspected ischemia in the anterior wall.

The pH response to the administration of blood cardioplegia through a newly constructed graft, and to the restoration of blood flow through the pedicle of a newly anastomosed left internal mammary artery, is a good indicator of the efficacy of the newly constructed grafts and the technical adequacy of the distal anastomoses. The restoration of tissue pH to normal levels in the anterior and posterior walls of the left ventricle, before weaning from cardiopulmonary bypass, is an indication of adequate revascularization.

Off-pump coronary artery bypass surgery has emerged over the past few years as a viable alternative to standard techniques using cardiopulmonary bypass and cardioplegic arrest. Multiple stabilization platforms are available to the surgeon, along with numerous techniques that aid in the exposure of the coronaries. Some platforms are based on the use of intracoronary shunting during the construction of the distal anastomoses, whereas others use the technique of coronary occlusion either with or without preconditioning. Selective distal perfusion techniques bail out the acutely ischemic myocardium. Standard hemodynamic and electrocardiographic monitoring, in addition to the use of continuous cardiac output and mixed venous monitoring, are the only tools currently available to detect the onset of regional and global ischemia that may occur with gross manipulation and positioning of the heart. The characteristics of the preferred embodiment myocardial pH monitoring system are a useful adjunct to the surgeon performing beating heart surgery. The probe size of the present invention renders it unobtrusive to the surgical field. The ability to move it and to reinsert it in different myocardial segments allows regional determinations of pH along specific vessel distributions. The ability to quantify regional ischemic changes with a rapid response time enables the surgeon to monitor the evolution of an ischemic episode and to modify the operative procedure so as to avoid the consequences of major ischemic insults. Modifications of the operative procedure can include the placement of an intracoronary shunt or perfusion of the vein grafts through an arterial inflow source before proceeding with the proximal anastomoses or, alternately, performing the proximal anastomoses first. The ability to detect ischemia with temporary coronary occlusion influences the sequence of performing the distal anstomoses. When global ischemia is encountered, assessed by progressive acidotic changes in both the anterior and posterior LV walls, the surgeon can decide to abort an "off-pump" procedure well before untoward consequences ensue.

Thus, online monitoring of myocardial tissue pH in the anterior and posterior walls of the left ventricle, using a preferred embodiment tissue pH electrode/monitoring system, provides a validated clinical tool for the real-time metabolic assessment of the magnitude of myocardial ischemia sustained in the course of a cardiac operation.

An improved 30-day and long-term postoperative outcomes have been observed when the integrated mean pH during the period of aortic clamping is kept at or above pH 6.8. Myocardial pH, when measured simultaneously in the anterior and posterior walls of the left ventricle, provides a good presentation of the global acid-base balance in the left ventricle myocardium.

In a particular embodiment, when the myocardial pH is only monitored without intervening to change it, it is observed that in 50% of the patients, the mean pH during the period of aortic clamping is less than 6.3 in either the anterior or the posterior wall. In contrast, in another particular embodiment, as pH-guided myocardial management is applied more than 60% of the patients have a mean pH during the period of aortic clamping of 6.8 or greater and have a better long-term postoperative outcome.

Clinically, optimal metabolic protection can be defined as the absence of severe myocardial tissue acidosis during the period of aortic occlusion as quantified by a temperature-corrected integrated mean pH of 6.8 or greater, which is predictive of a favorable postoperative outcome.

Myocardial tissue pH measurements are corrected for temperature in two ways. The physical effect of temperature on the slope of the pH electrode is corrected by the use of the Nernst equation in the course of the calibration of the pH electrode. To account for the physiologic change in pH with temperature, a correction factor of 0.017 pH units/°C is used to generate plots of pH corrected to 37°C, thus allowing for a comparative assessment of the acid/base state of the myocardium independent of the effect of changes in myocardial temperature. Integrated mean myocardial tissue pH and myocardial temperature values are calculated for the period of aortic occlusion by planimetry of the myocardial tissue pH and myocardial temperature plots during that period, as described previously.

In accordance with a preferred embodiment, the on-line pH monitoring system is an alternating current (AC)-powered, microprocessor-based monitor with two measuring probes and a reference electrode with a common connector that plugs into a junction box that is attached to the monitor. The two pH probes and reference electrode are called the pH sensor. Continuous pH and temperature measurements are obtained from one or both probes and displayed on the monitor. The sensor is made sterile, by gamma radiation sterilization.

As described hereinbefore, the pH probe consists of a closed end glass tube made from pH sensitive glass. The tube is filled with an electrolyte (KCl) solution in which a silvered wire coated with silver chloride is inserted. The wire is attached to a cable that is encased in an electrically shielded sheath and attaches to the monitor. The tip of the glass is pointed to allow easy insertion into the myocardial tissue during use. The thermistor has a metal oxide ceramic tip which is imbedded in the plastic surrounding the rear of the glass tube.

A reference electrode is used to complete the circuit. The reference consists of a Ag/AgCl wire inserted into a flexible plastic tube of KCl electrolyte solution. The front end of the tube is tapered to a small diameter to facilitate insertion into the tissue (usually near the sternum) during use. It is plugged with a semipermeable material that prevents bulk leakage of fluid but maintains electrical contact with the patient during pH measurements. The wire protrudes from the sealed back end of the tube and is attached to a cable which connects to the monitor.

The analog voltage signal from the probes is fed into the junction box that amplifies and conditions the analog signal to remove any interfering noise. The analog signal is then converted into digital information in the analog to digital converter. This digital signal is then fed from the junction box to the monitor where a conversation algorithm, in the monitor, is used to convert digital information into pH units. The values are then displayed on the monitor screen.

The monitor for use with the pH monitoring system consists of a single board computer and a dedicated circuit that contains digital circuitry to interface with the junction box that connects to the sensor. The monitor has a liquid crystal display (LCD) flat touch screen display with user interface buttons that control the mode and the operation of the monitor. The pH system is line powered and only the data from the case is stored to memory. There is a printer that enables the user to print out the case results. The monitor can be mounted on a heart pump pole, an intravenous (IV) pole, or rest on a flat surface.

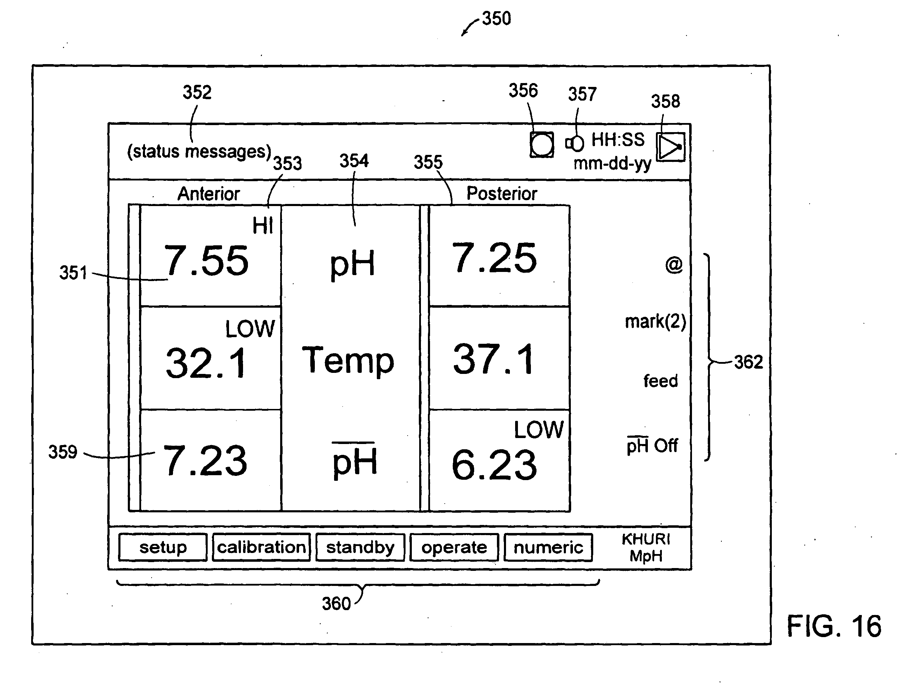

Systems and software are developed in conjunction with Terumo Cardiovascular Systems located at Ann Arbor, Michigan. Figure 16 illustrates the front panel of the user interface monitor with numerical values on the screen, in accordance with a preferred embodiment of the present invention. Note, the display screen reflects the values and alarms. The no stripe prose values 351 display shows values on the screen which are correlated with the cable with no stripe on the pH probes. The status bar 352 displays user messages regarding printer status, probe status, calibration status, flash car status, and alarms status.

The alarm indicators 353 display numbers that are outside the user defined limits that set off alarms and within the text box, a "HI" or "LOW" indicator is displayed. The stripped probe values 355 correlate with the cable stripe on the pH probes. The value labels 354 are pH, ' , ∼ and Temp. The probe icon 356 indicates the monitor is connected to the junction box and the software. The alarm icons 357 indicate the display status of the alarm (on/off). The heart beat icons 358 in the status bar indicate to the user that the software is operational. The inactive values 359 are also displayed on the user interface screen. The system mode buttons 360 are used to select between different modes (active, calibrate, standby and operate) and the label for the operate mode display choice (numeric and graphical). The soft buttons 362 are mode specific and are software driven function buttons. Their purpose can vary from screen to screen.

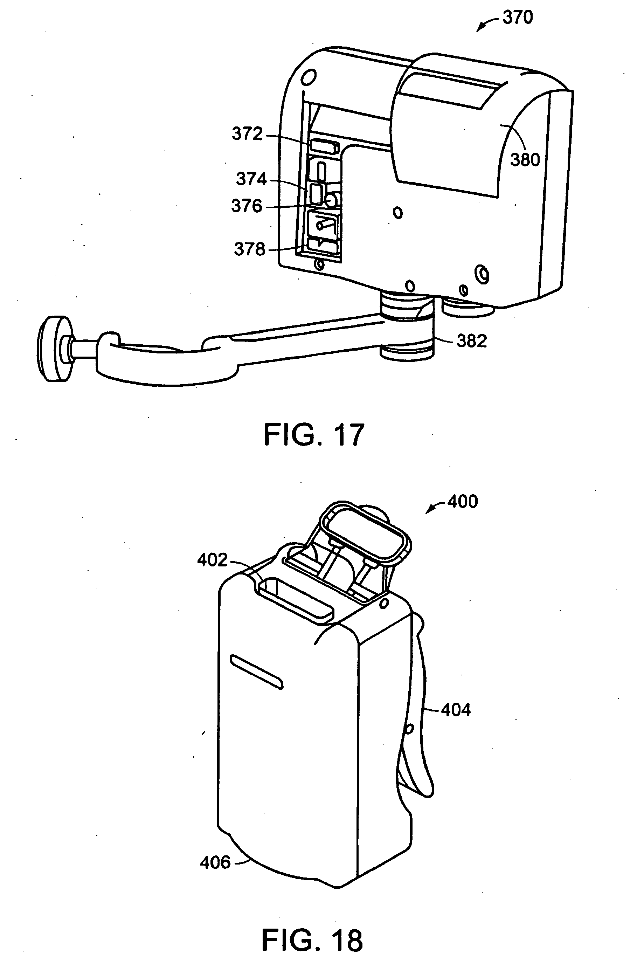

Figure 17 illustrates the back view of a monitor included in the preferred embodiment of the present invention pH monitoring system. The data output port 372 allows serial transmission of the pH values to an external computer or data acquisition device. The system power switch 374 turns the power to the monitor on or off. When the monitor is turned off, the most recent calibration values and setup parameters are saved in memory. After the user turns the monitor back on, these values are automatically recalled. The monitor to junction box connector 376 is a circular connector coupling the monitor to the junction box. The power cord connector 378 is the receptacle for the power cord that connects to an AC power supply. The power cord supplies the monitoring system with a hospital grade AC cord. The printer cover 380 protects the printer and paper from spills. Further, the monitor pole clamp 382 allows easy mounting (and dismounting) of the monitor to the monitor pole clamp tray.

Figure 18 illustrates the junction box 400 in accordance with a preferred embodiment of the present invention. The junction box sensor connector 402 is a receptacle for the sensor cable connector. The drape clip 404 allows the junction box to clip onto the drape cloth. The junction box to monitor connector 406 is a circular connector for the monitor to junction box coupling.

The sensor in accordance with a preferred embodiment consists of two pH measurement probes and a reference electrode with a common jack that plugs into the junction box, which is attached to the monitor. The measurement probes are designed with a pointed tip for insertion into tissue with minimal resistance, and a right angle bend about 5/8" from the tip to prevent over penetration into the myocardial tissue. Both pH probes contain a thermistor to measure the local tissue temperature. The temperature measurement is used to calculate any temperature correction to the probe, which converts the measured pH values to a corresponding 37 degrees corrected value. The reference electrode consists of an immobilized buffer in intimate contact with silvered wire. There is no thermistory on the reference electrode. The probes are distinguished from one another with a stripe along the cable. This cable that attaches the two probes and reference electrode to the monitor is a six-foot long electrical shielded flexible cable.

Both pH measurement probes can be packaged immersed in a calibrant buffer solution. This solution stabilizes the microsensors during storage and is used during the two point calibration to establish predictable pH values.

Each sensor is intended for a single use. An aseptic method is used to protect from contamination. The sensor remains sterile as long as the package is unopened and undamaged. Each sensor is individually packaged in an inner foil pouch, and then a clear outer pouch and has a recommended shelf life indicated by the lot number expiration date printed on each.

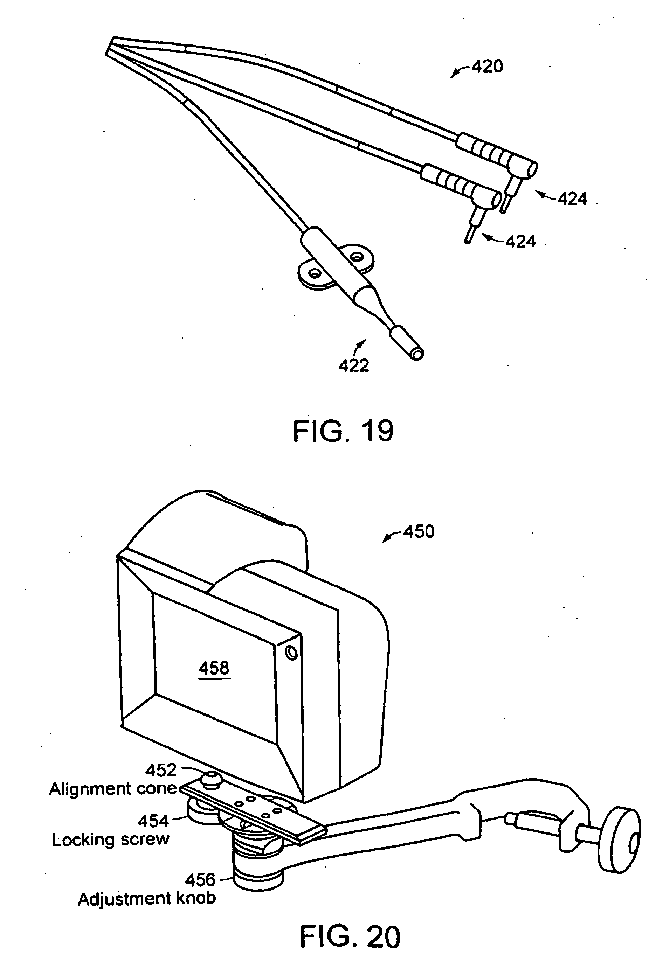

Figure 19 illustrates the sensing end 420 of the sensor used in the pH monitoring system in accordance with the present invention. There is a reference electrode 422 and at least two pH probes 424.

Figure 20 illustrates the attachment of a pole clamp and the user interface monitor in accordance with a preferred embodiment of the pH monitoring system. The pole clamp for use with the system attaches to a standard heart-lung machine pole, an intravenous (IV) pole or can rest on a flat surface. The pole clamp consists of an arm and a tray with an alignment cone for easy mounting and dismounting. Once the monitor is placed on the tray, the locking screw is pushed up towards the monitor and tightened in a clock-wise direction to secure the monitor to the monitor pole clamp.

A two point calibration of the sensor in a preferred embodiment includes the steps of connecting the sensor connector into the junction box, removing a cap from the reference electrode and inserting into the calibration cuvette per operating use instructions, rinsing sensor probes off and storing in saline solution. Further, the steps include connecting the sensor assembly connector to the junction box, and setting the monitor to calibrate mode.

Sensor insertion in a preferred embodiment includes removing the sensor probes from the rinse/storage solution and inserting them into the myocardial tissue. Further reference electrode is removed from the risen/storage solution and inserted through a small incision into tissue, e.g. chest cavity.

The monitor 458 is made operational by setting the monitor to "Operate" mode. The following display options are selected: change the display modes for viewing data (choices are "numeric" or "graphic")>, mark or print data, change patient temperature mode and stop or stop 'i Integration. To set up the monitor, the following steps are used including attaching the monitor pole clamp securely to a standard heart-lung machine pole, or an IV pole and tightening the pole clamp until it's secure. Further, the monitor is attached to the tray of the pole clamp and the monitor is placed on the tray, positioned correctly on the alignment cone. The locking screw on the bottom of the tray is used to secure the tray is used to secure the tray.

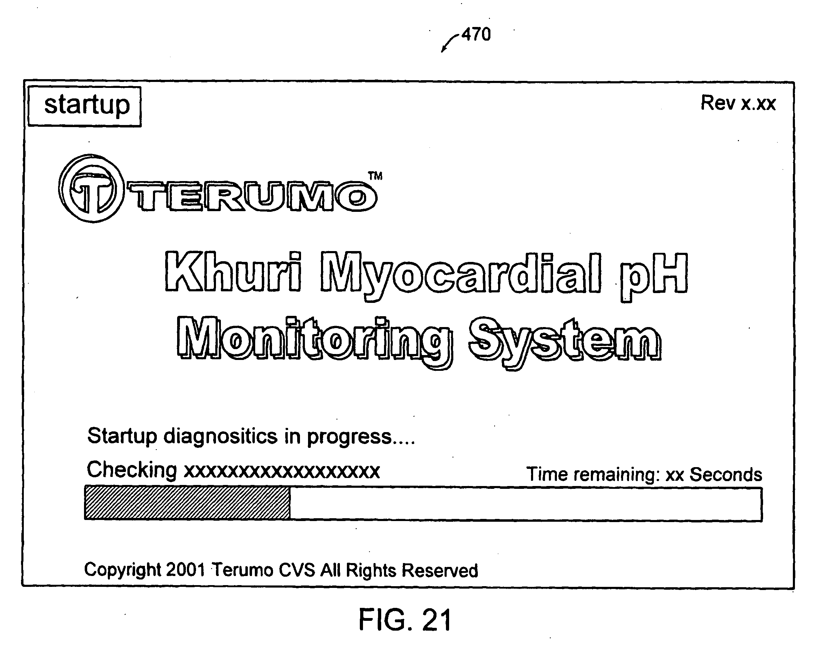

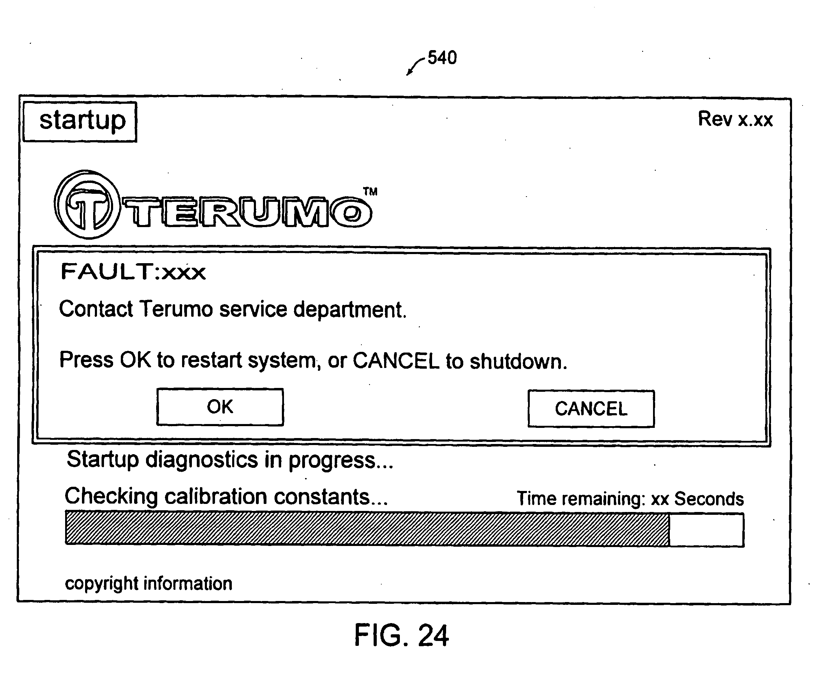

Figure 21 illustrates a display screen 470 of a user interface monitor in accordance with a preferred embodiment of the present invention. Once the monitoring system is operable, the system diagnostics tests to run automatically. These tests verify the function of the monitor and junction box electronics, check the flash card and flash card memory, and verifies the junction box calibration data. The startup sequence begins, and the system diagnostic testing takes about 40 seconds in a preferred embodiment. A time bar under the startup message indicates the progression of tests. If an error is detected during the system diagnostics, an error message appears in the center of the screen as illustrated in the display screen 540 in Figure 24.

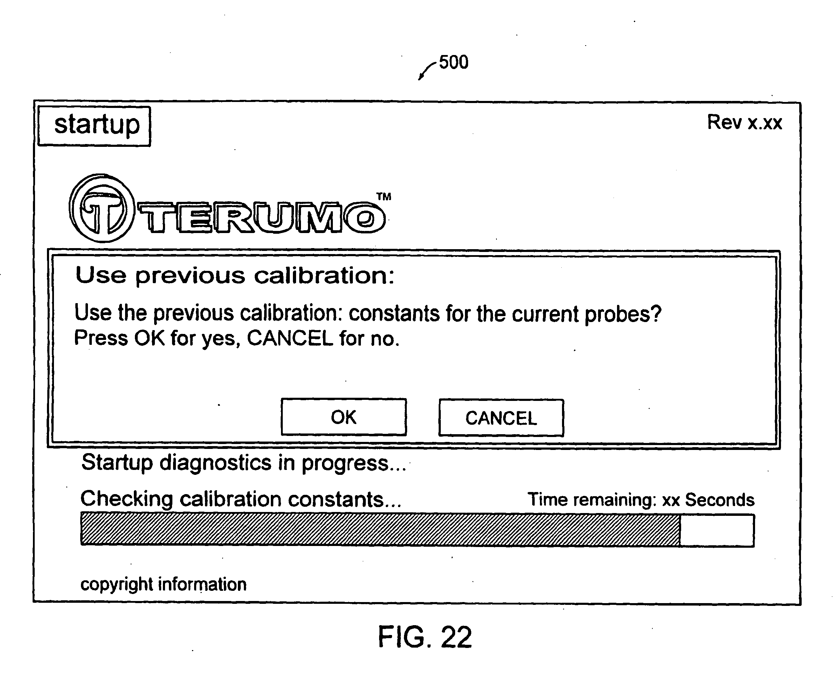

Figure 22 illustrates a display screen 500 of the user interface monitor in accordance with the present invention querying the use of the previous calibration. In particular, if a sensor has been calibrated with in the last 8 hours, for example, the system prompts the user if an earlier calibration can be used.

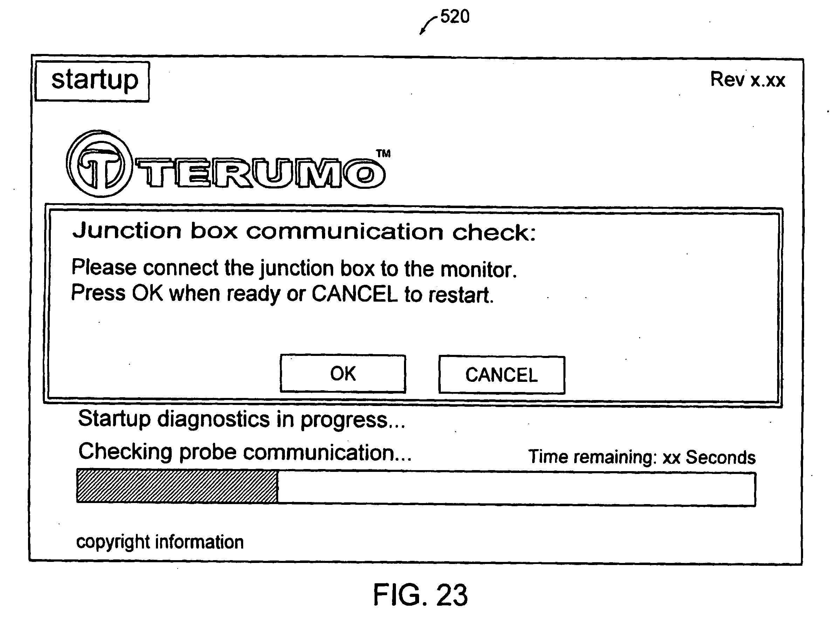

Figure 23 illustrates the display screen 520 of the user interface monitor notifying the user about a communication check in accordance with a preferred embodiment. Screen 520 is displayed if the junction box has not been connected properly to the monitor. Errors that are recoverable offer an option to continue by pressing the OK button. Any consequences of continuing despite the error are noted in the error message.

In accordance with a preferred embodiment, system settings are customized. The following is a list of the setup screens that a user can interface with: printer options screen for choosing options for how and when the user wants to print pH, ' and temperature values, an alert screen for setting high and low parameter thresholds to alert the user when patient pH and temperature parameter values fall outside the limits the user specifies, a graphic display screen for choosing which values to see displayed in graphical format during operations, a general screen for choosing general screen parameters; which language the monitor needs to display, the current date, time, date format, and more, a calculations screen with no parameters and a serial port configuration screen for setting parameters for sending data to a computer or data acquisition system.

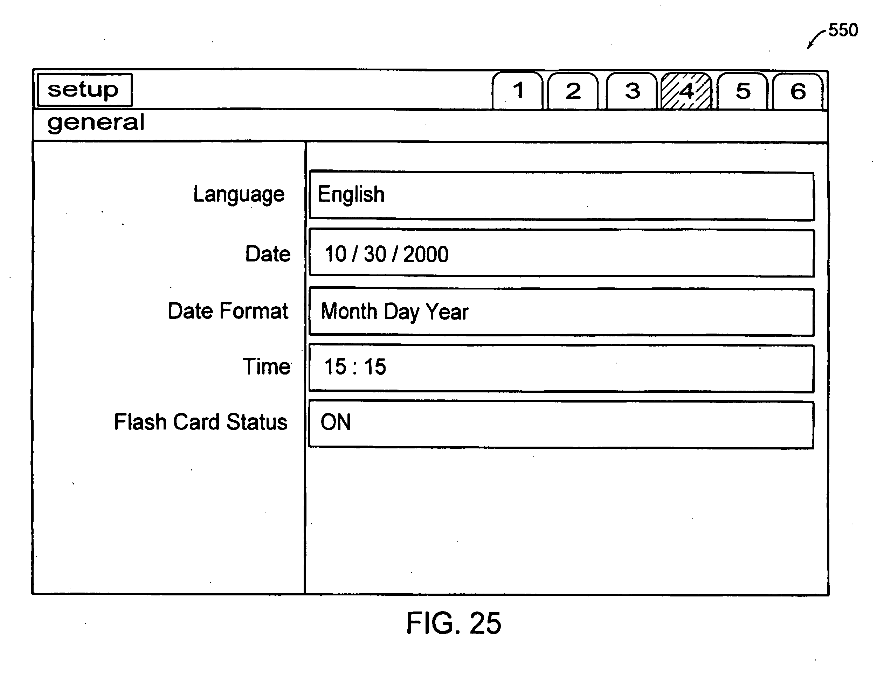

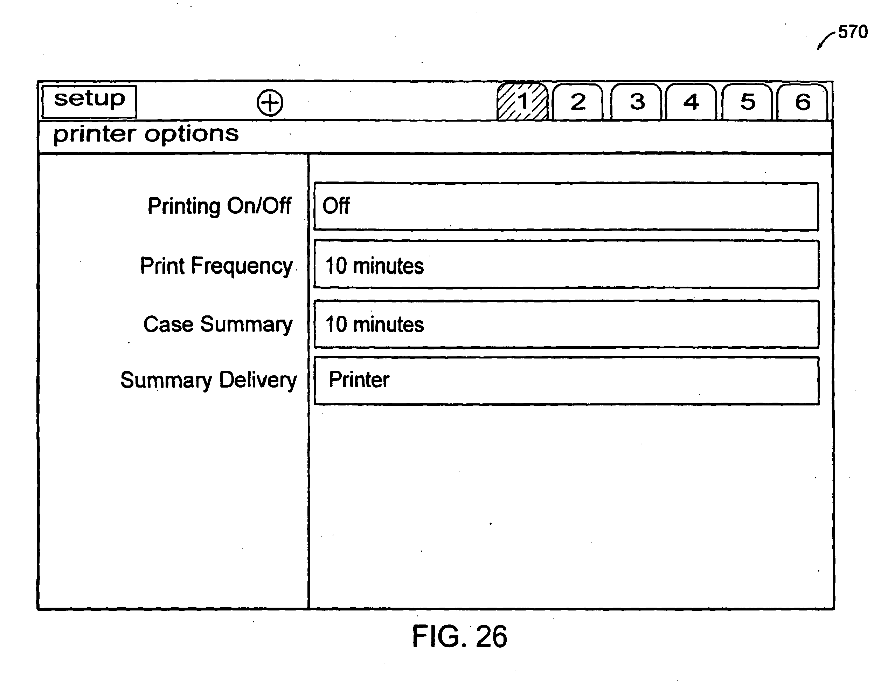

To choose options on the setup screens, the tab buttons at the top of the screen are tapped to select one of six individual setup screens,. When the tab button is tapped, the old tab button is unhighlighted, and the new button is highlighted to indicate the current tab. Figure 25 illustrates a display screen 550 of the user interface monitor and displays the general screen in accordance with a preferred embodiment of the present invention. Figure 26 illustrates a display screen 570 of the user interface monitor in accordance with a preferred embodiment that details the printer options.

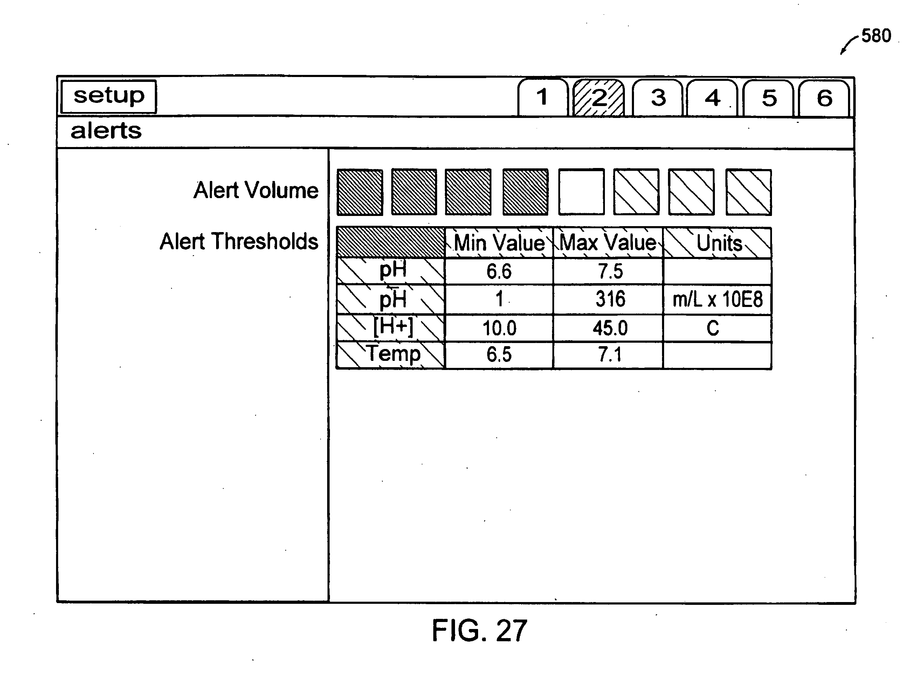

Figure 27 illustrates the display screen 580 of the user interface monitor that displays the set up of the alerts in accordance with the preferred embodiment. The second screen in setup mode the alert screen which allows the user to set alerts that notify the user about possible problems during a procedure. On this screen, the acceptable ranges for a patient's pH and temperature parameters and the volume of the alert that sounds when a patient's value fall outside the acceptable range are set.

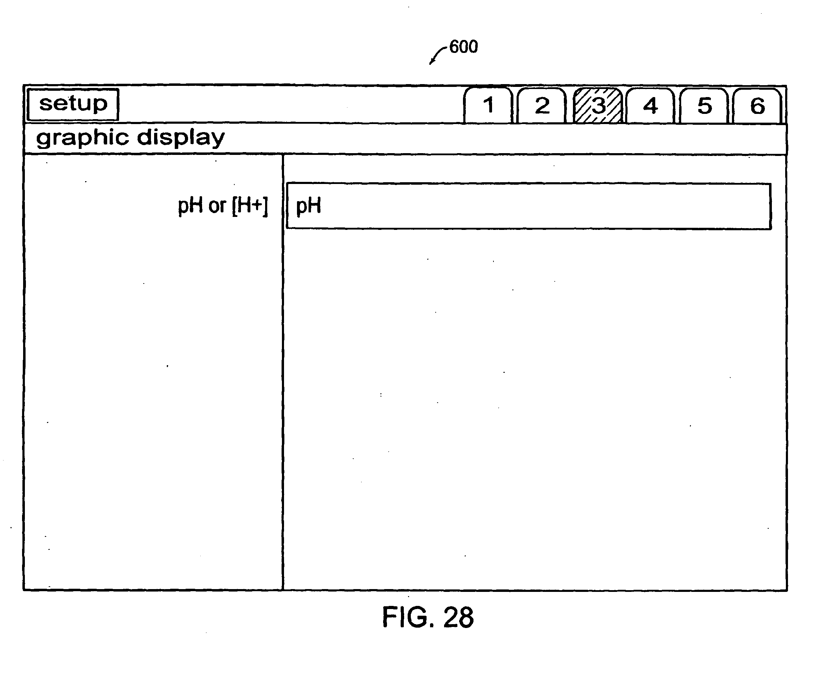

Figure 28 illustrates a display screen 600 of a user interface for choosing graphic displays in accordance with a preferred embodiment of the present invention. In the operate mode, the monitoring pH system can display the patient's pH or ∼ values in a graphical format. The graph shows values over two selectable time periods, so changes in a patient's values can be observed at a glance. The third screen in setup mode, the graphic display screen, allows the user to toggle between pH or ∼ values to display in the graphical format. On the graphic display setup screen, the selection and edit keys are displayed as tab edit, tab the value button and select either pH or ∼, tab edit again to accept your selection.

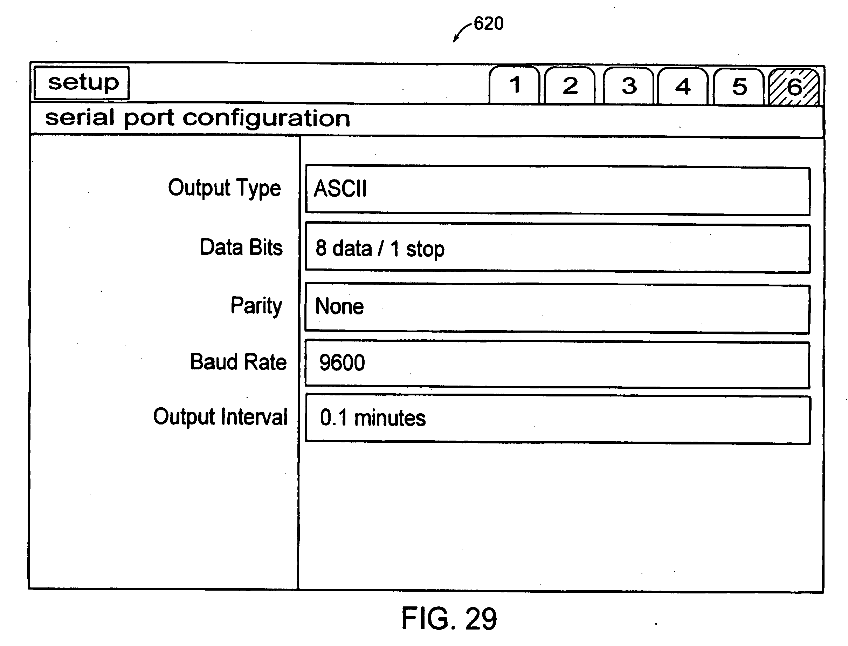

Figure 29 illustrates a display screen 620 of a user interface for setting parameters for communicating with an external device in accordance with a preferred embodiment of the present invention. This screen, the serial port configuration screen, is used to set parameters for communicating with an external serial printer, computer, or data acquisition system. When attached to an external device, the system can send operational information and pH, ', ∼ or temperature values at a prescribed frequency or on demand.

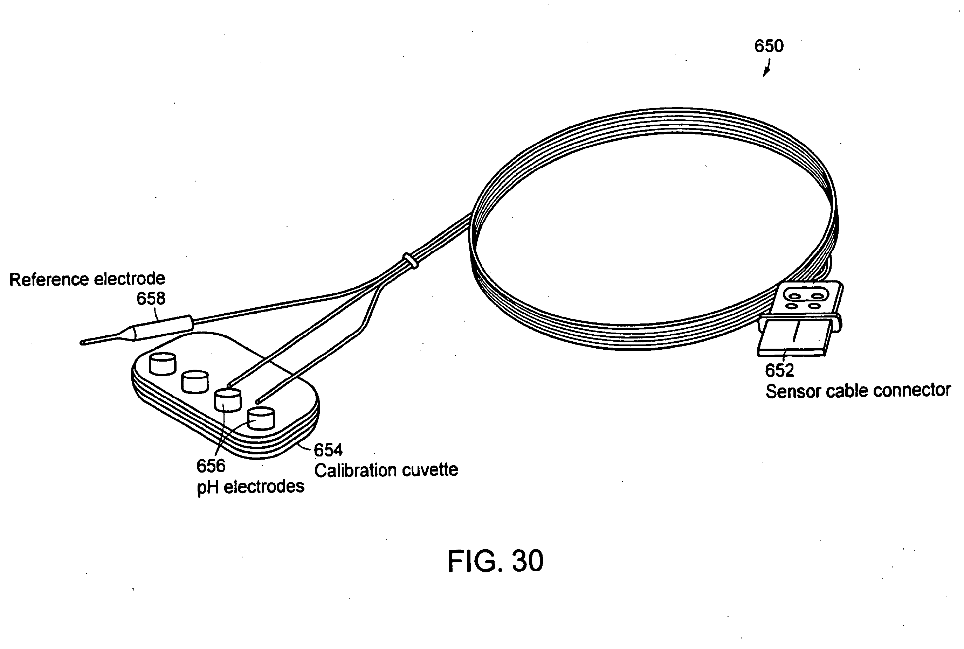

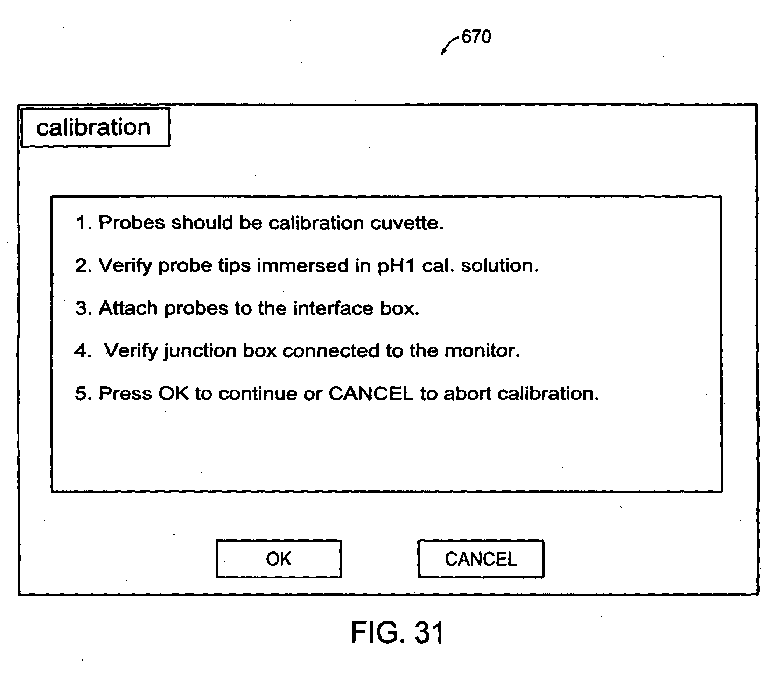

Figure 30 illustrates a view of the monitoring pH sensor being calibrated in accordance with a preferred embodiment. For the two point calibration of the sensor used in the pH monitoring system it is first verified that the sensor and the monitor to junction box cables are securely in place. The disposable sensor assembly 650 is supplied with the two pH probes 656 submerged in one of two calibrants contained in a calibration cuvette 654. The reference electrode 658 is supplied stored dry and the user inserts the reference electrode into the calibration cuvette. Figure 31 illustrates the display screen 670 of the user interface further detailing the method of calibration in a preferred embodiment.

This calibration cuvette 654 consists of two sealed compartments, each compartment containing two different pH buffer solutions. One compartment is used as the pH probes' storage/hydration medium. The other compartment has three thin membranes that can be easily punctured when transferring the pH probes and reference electrode into the second calibration point. The calibration in a preferred embodiment consists of measuring the voltage with the pH probes and reference electrode in contact with the first calibration buffer. The user then transfers the two pH probes and the reference electrode to the second calibration buffer compartment. After calibration, probes can be stored in the second calibration buffer before use. The probe can be used within 3 hours after calibration.

While in the standby mode, the setup and calibrate buttons are enabled and the operate button is enabled if the probes have been calibrated. At any time during these modes the user can access the standby mode. In the standby mode the "shutdown" button is tapped to close all applications, exit windows and the processor is in the low power mode. The "history" button is tapped to find out all of the data files stored on the flash card. The user can also select from a list a history case file to see the case graph or print the selected file. The "print case" in the standby mode is tapped to send the last case to the printer/serial port/or both at the time interval specified (both, dependent on the setup mode's printer options selections for "summary Delivery" and "Print Frequency"). The system information button displays the following statistics: monitor serial number, junction box serial number, Windows CE version, mpH software version, mpH build number and number of boots.

The method to insert the probe includes the removal of the two pH probes from the rinse/storage solution and insertion into the myocardial tissue about 5/8" deep, up to the right angle bend of the probe tip. The insertion location must be made in an area of the heart that is thick enough to prevent the probe from penetrating into the cavity of the heart, thus coming in direct contract with the blood.

The probes and the reference electrode can be sutured into place. The probes and the reference electrode can be removed and reinserted without recalibrating the system in a preferred embodiment.

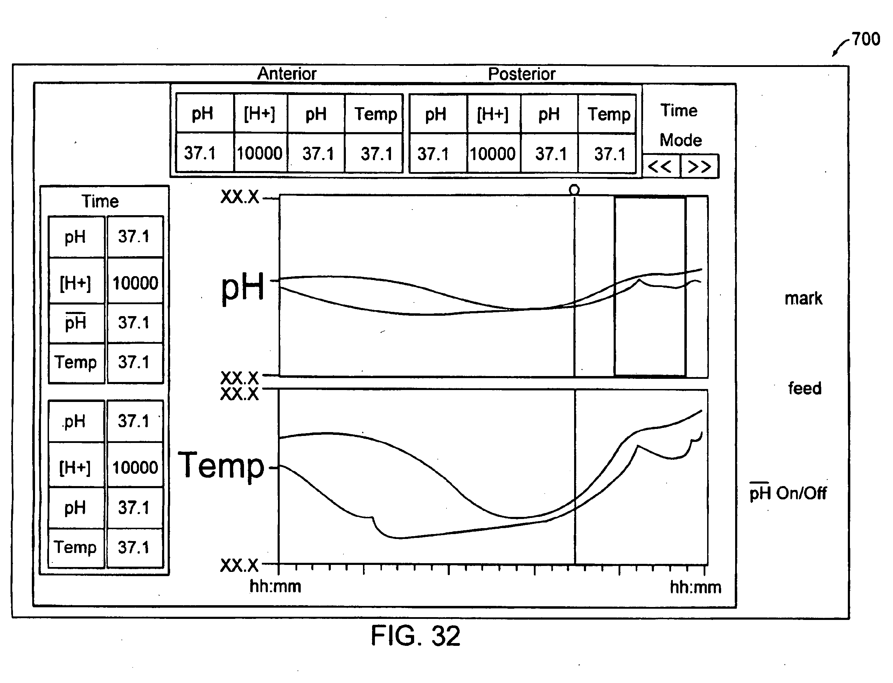

The display screen 700 shown in Figure 32 illustrates the survival modes in a preferred embodiment. The numeric display can be set to provide in an alternate preferred embodiment no alarms. The "mark" button increments a mark counter which starts at "1" for each new case. After the first tap of this button, the mark counter value is displayed in the button text of the form: Mark (x), where x = value of the mark counter. The mark counter is stored in the history file for the case. The button is disabled until the value is stored in the file. The "feed" button advances the printer paper. The "'On/Off" button toggles between "Start Integration" and "Stop Integration". During the integration period (after the start button is tapped), the screen updates the " " by integrating the values from when the start button was tapped. When stop button is tapped, the " ' " field will be fixed with the last value and changes color to indicate that the numbers are not be updated. During a procedure the user can switch between two display modes --- numeric and graphic. Continuous pH and temperature (37 degree calculations) values are shown in each display mode. On the graphic display, the "(left) and .. (right) arrow keys move the cursor through the graph, and display the data values in the history window referenced by the position of the cursor. Alternatively, the user can touch an area of the graph, and the cursor jumps to that point (as long as data is present at the selected point).

Two graphs are displayed in the graphic mode. The top graph is a user-defined selection set in the setup screen. The bottom graph is the temperature data from both probes. Data is added from left to right, oldest values on the left side of the graph, newest values to the right. When the graph is full, the entire graph scrolls left, creating room for a new data point at the right most position on the graph. Current values are displayed on the left side of the screen, and are updated at the history data rate (10Hz). Historic values are displayed above the graph and reflect the values where the cursor is located. Out of range numbers are dashed, and values that cause an alarm have a background.

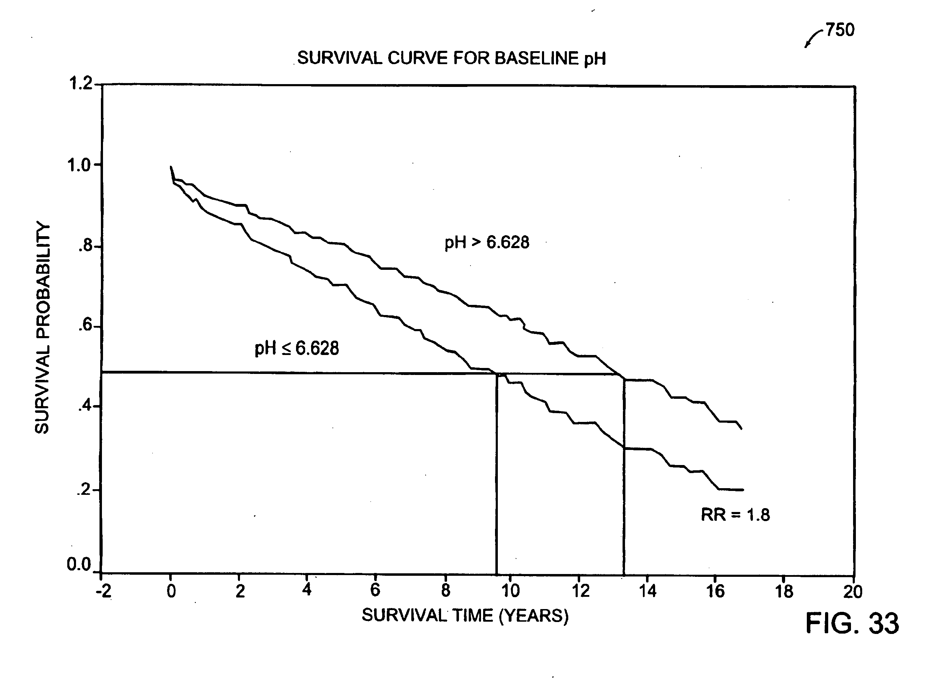

Figure 33 graphically illustrates the survival curve 750 for the baseline pH which is defined as the pH measured just prior to insertion of an aortic clamp in accordance with a preferred embodiment of the present invention. The effect of intraoperative acidosis on long-term survival following cardiac surgery in accordance with a preferred embodiment of the present invention was monitored over an average of a 10-year period, for a range of 3-17 years. The statistical analysis in accordance with the preferred embodiment included Cox proportional hazards regression analysis which provided a dependent variable that included survival as a continuous variable. Further statistical analysis included the automatic interaction detection analysis that included as survival as a dichotomous variable and a 30-day post-operative deaths censured as an additional statistical parameter.

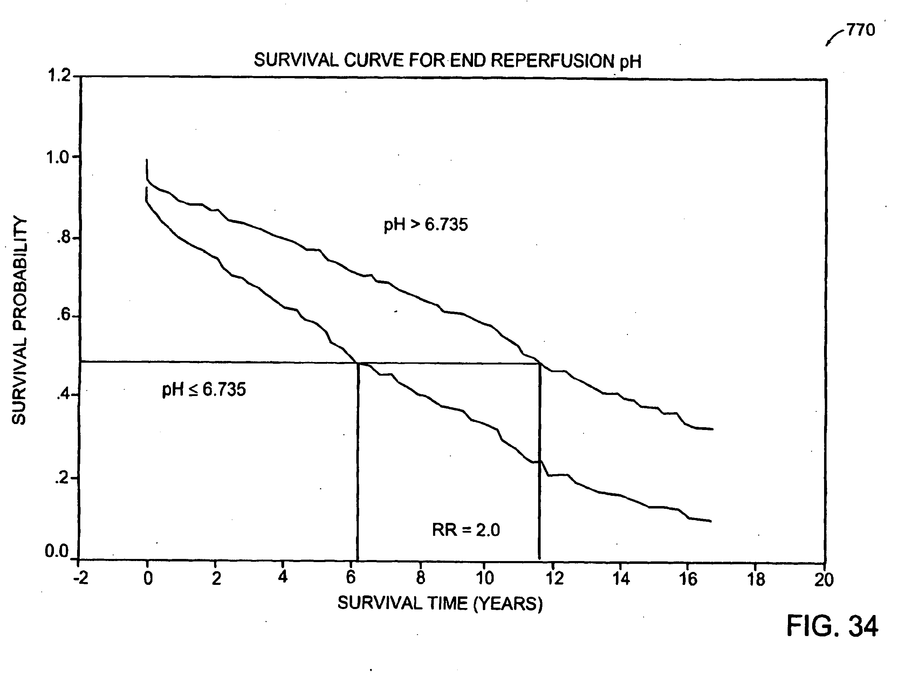

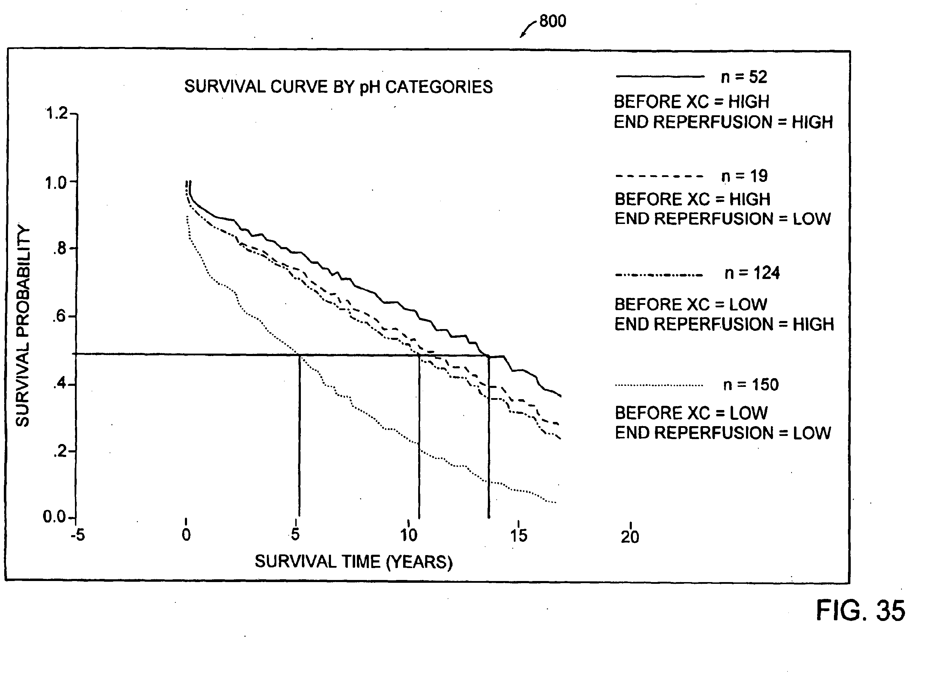

Figure 34 graphically illustrates the survival curve 770 in patients based on the pH at the end of the reperfusion in accordance with a preferred embodiment of the present invention. Figure 35 graphically illustrates the survival curves 800 for different baseline and end reperfusion pH categories. The variable most significant by Cox regression analysis is the lower of the anterior wall or posterior wall pH at the end of reperfusion. These curves show that end reperfusion pH below 6.735 results in almost a 50% decrease in long-term survival and that raising the pH in the course of an operation from a baseline below 6.628 to an end reperfusion pH > 6.735 improved the long-term survival by a factor of two.

While this invention has been particularly shown and described with references to preferred embodiments thereof, it will be understood by those skilled in the art that various changes in form and details may be made therein without departing from the scope of the invention encompassed by the appended claims.

## Claims

1. A tissue monitoring system **characterized by**
a first pH electrode (136) disposed in an anterior wall of a ventricle;
a second pH electrode (422) disposed in a posterior wall of said ventricle, the electrodes measuring the pH of the tissue during cardiac surgery;
a temperature sensor that measures temperature of the tissue;
a processor (160) that determines tissue pH in response to a pH electrode measurement and a temperature measurement;
such that the processor determines if the tissue pH falls below a threshold level indicative of acidosis with the first and second electrode and determines a post-operative outcome based on the threshold level and the tissue pH measured by the first and second electrode;
a display (12) connected to the processor that displays monitoring operations; and
a fluid delivery system (140) having a manifold connected to a fluid source (144) to control fluid delivery to a plurality of sites and having a probe (148, 150, 152) to be inserted into at least one site.

2. The system of claim 1,
a) wherein the pH electrode contacts tissue of a patient such that pH data of the tissue is monitored to determine if tissue pH deviates from a threshold level indicative of ischemia, or
b) further comprising a controller (158) that is connected to the fluid delivery system, and optionally wherein the controller controls the flow rate of a preservation solution delivered to a surgical site, or the controller controls temperature of a preservation solution, or the controller controls a site of delivery of a preservation solution.

3. The system of claim 1
a) further comprising a catheter which delivers the fluid, or
b) wherein the delivery system applies coronary artery pressure on a proximal portion of an artery, or
c) further comprising a balloon catheter, or
d) further comprising a balloon of a retrograde coronary sinus catheter, or
e) wherein the pH electrode is positioned relative to cardiac tissue of a patient with a percutaneous catheter, or
f) wherein the pH electrode is delivered to a tissue site of a patient using a laparoscope, or
g) wherein the pH electrode is positioned using an endoscope.

4. The system of claim 1 wherein the threshold level indicative of adverse acidosis during a period of aortic clamping is below 6.8.

5. The system of claim 1 further comprising a controller (158) that controls acidosis by delivering a preservation solution to a heart at a plurality of sites, and optionally wherein the controller alters the flow rate of the preservation solution, or the controller alters the temperature of the preservation solution, or the controller alters the site of delivery of the preservation solution.

6. The system of claim 1
a) further comprising a tip of a catheter which delivers a solution, or
b) further comprising means for applying direct coronary artery pressure on a proximal portion of the artery, or
c) further comprising means for occluding an orifice of a left main coronary artery, or
d) further comprising means (170) for inflating a balloon (172) of a retrograde coronary sinus catheter, or
e) further comprising means for administering a bolus of preservation solution through an orifice of a right coronary artery, or
f) wherein the processor calculates an integrated mean value of pH measured with a first and a second electrode.

## Patentansprüche

1. Gewebeüberwachungssystem, **gekennzeichnet durch**
eine erste pH-Elektrode (136), angeordnet an einer vorderen Wand eines Ventrikels, eine zweite pH-Elektrode (422), angeordnet an einer hinteren Wand des Ventrikels, wobei die Elektroden den pH des Gewebes während Herzchirurgie messen,
einen Temperatursensor, der die Temperatur des Gewebes misst,
einen Prozessor (160), der den Gewebe-pH als Antwort auf eine pH-Elektrodenmessung und eine Temperaturmessung bestimmt,
derart, dass der Prozessor mit der ersten und zweiten Elektrode feststellt, wenn der Gewebe-pH unter einen Grenzwert fällt, der indikativ für Azidose ist, und einen postoperativen Erfolg feststellt, basierend auf dem Grenzwert und dem Gewebe-pH, gemessen **durch** die erste und zweite Elektrode,
ein Display (12), angeschlossen an den Prozessor, welches die beobachteten Arbeitsabläufe anzeigt und
ein Flüssigkeitszufuhrsystem (140) mit einem Verteiler, der an einer Flüssigkeitsquelle (144) angeschlossen ist, um Flüssigkeitslieferung an eine Vielzahl von Stellen zu steuern, und der eine Sonde (148, 150, 152) hat, die in zumindest eine Stelle eingesetzt wird.

2. Das System nach Anspruch 1,
a) wobei die pH-Elektrode Gewebe eines Patienten so kontaktiert, dass pH-Daten des Gewebes überwacht werden, um festzustellen, wenn der Gewebe-pH von einem Grenzwert abweicht, der für Ischämie indikativ ist, oder
b) weiterhin aufweisend einen Regler (158), der an das Flüssigkeitszufuhrsystem angeschlossen ist und wobei wahlweise der Regler die Fließgeschwindigkeit einer Erhaltungsflüssigkeit steuert, die an eine Operationsstelle geliefert wird, oder der Regler die Temperatur einer Erhaltungsflüssigkeit steuert, oder der Regler eine Stelle der Zufuhr einer Erhaltungsflüssigkeit steuert.

3. Das System nach Anspruch 1,
a) weiterhin aufweisend einen Katheter, der die Flüssigkeit liefert, oder
b) wobei das Zufuhrsystem koronaren Arteriendruck auf einen nahen Abschnitt einer Arterie ausübt, oder
c) weiterhin aufweisend einen Ballonkatheter oder
d) weiterhin aufweisend einen Ballon eines retrograden koronaren Sinuskatheters, oder
e) wobei die pH-Elektrode relativ zu dem Herzgewebe eines Patienten mit einem perkutanen Katheter positioniert wird, oder
f) wobei die pH-Elektrode unter Verwendung eines Laparoskops an eine Gewebestelle eines Patienten geliefert wird, oder
g) wobei die pH-Elektrode unter Verwendung eines Endoskops positioniert wird.

4. Das System nach Anspruch 1, wobei der Grenzwert, der für unerwünschte Azidose indikativ ist, während eines Zeitraums des Abklemmens der Aorta unterhalb von 6,8 ist.

5. Das System nach Anspruch 1, weiterhin aufweisend einen Regler (158), der Azidose reguliert, indem eine Erhaltungsflüssigkeit in ein Herz an eine Vielzahl von Stellen geliefert wird und wobei wahlweise der Regler die Fließgeschwindigkeit der Erhaltungsflüssigkeit verändert oder der Regler die Temperatur der Erhaltungsflüssigkeit verändert oder der Regler die Stelle der Lieferung der Erhaltungsflüssigkeit ändert.

6. Das System nach Anspruch 1
a) weiterhin aufweisend eine Spitze eines Katheters, der eine Lösung liefert oder
b) weiterhin aufweisend Vorrichtungen zum Aufbringen von direktem koronarem Arteriendruck auf einen nahen Abschnitt der Arterie, oder
c) weiterhin aufweisend Vorrichtungen zum Verschließen einer Öffnung einer linken Hauptkoronararterie, oder
d) weiterhin aufweisend Vorrichtungen (170) zum Aufblasen eines Ballons (172), eines retrograden koronaren Sinuskatheters, oder
e) weiterhin aufweisend Vorrichtungen, um einen Bolus von Erhaltungsflüssigkeit durch eine Öffnung einer rechten Koronararterie zu verabreichen, oder
f) wobei der Prozessor einen integrierten Mittelwert eines pH berechnet, der mit einer ersten und einer zweiten Elektrode gemessen wurde.

## Revendications

1. Système de suivi tissulaire **caractérisé par**
une première électrode de pH (136) disposée dans une paroi antérieure d'un ventricule,
une seconde électrode de pH (422) disposée dans une paroi postérieure dudit ventricule, les électrodes mesurant le pH du tissu pendant une chirurgie cardiaque,
un capteur de température qui mesure la température du tissu,
un processeur (160) qui détermine le pH tissulaire en réponse à une mesure d'électrode de pH et à une mesure de température,
de sorte que le processeur détermine si le pH tissulaire chute au-dessous d'un niveau de seuil indicateur d'acidose à l'aide des première et seconde électrodes, et détermine un résultat post-opératoire en fonction du niveau de seuil et du pH tissulaire mesuré par les première et seconde électrodes,
un affichage (12) relié au processeur qui affiche des opérations de suivi, et
un système de délivrance de fluide (140) ayant un collecteur relié à une source de fluide (144) pour commander une délivrance de fluide vers une pluralité de sites, et ayant une sonde (148, 150, 152) devant être insérée dans au moins un site.

2. Système selon la revendication 1,
a) dans lequel l'électrode de pH est en contact avec le tissu d'un patient de telle sorte que des données de pH du tissu sont suivies afin de déterminer si le pH tissulaire s'écarte d'un niveau de seuil indicateur d'ischémie, ou
b) comportant en outre un contrôleur (158) qui est relié au système de délivrance de fluide, et facultativement dans lequel le contrôleur commande le débit d'écoulement d'une solution de préservation délivrée à un site chirurgical, ou le contrôleur commande la température d'une solution de préservation, ou le contrôleur commande un site de délivrance d'une solution de préservation.

3. Système selon la revendication 1
a) comportant en outre un cathéter qui délivre le fluide, ou
b) dans lequel le système de délivrance applique une pression d'artère coronaire sur une partie proximale d'une artère, ou
c) comportant en outre un cathéter à ballonnet, ou
d) comportant en outre un ballonnet d'un cathéter rétrograde de sinus coronaire, ou
e) dans lequel l'électrode de pH est positionnée par rapport au tissu cardiaque d'un patient à l'aide d'un cathéter percutané, ou
f) dans lequel l'électrode de pH est délivrée à un site tissulaire d'un patient en utilisant un laparoscope, ou
g) dans lequel l'électrode de pH est positionnée en utilisant un endoscope.

4. Système selon la revendication 1 dans lequel le niveau de seuil indicateur d'acidose néfaste pendant une période de clampage aortique est en dessous de 6.8.

5. Système selon la revendication 1 comportant en outre un contrôleur (158) qui commande l'acidose en délivrant une solution de préservation à un coeur à une pluralité de sites, et facultativement dans lequel le contrôleur modifie le débit d'écoulement de la solution de préservation, ou le contrôleur modifie la température de la solution de préservation, ou le contrôleur modifie le site de délivrance de la solution de préservation.

6. Système selon la revendication 1
a) comportant en outre une extrémité d'un cathéter qui délivre une solution, ou
b) comportant en outre des moyens pour appliquer une pression d'artère coronaire directe sur une partie proximale de l'artère, ou
c) comportant en outre des moyens d'occlusion d'un orifice d'une artère coronaire principale gauche, ou
d) comportant en outre des moyens (170) pour gonfler un ballonnet (172) d'un cathéter rétrograde de sinus coronaire, ou
e) comportant en outre des moyens pour administrer un bolus de solution de préservation à travers un orifice d'une artère coronaire droite, ou
f) dans lequel le processeur calcule une valeur moyenne intégrée d'un pH mesuré à l'aide de première et seconde électrodes.
